# EUROPEAN PATENT APPLICATION

(11) **EP 1 865 320 A2**
(43) Date of publication of application: **12.12.2007**
(21) Application number: 07252322.8
(22) Date of filing: 08.06.2007
(51) Int. Cl.: G01N 33/50, G01N 33/74, C07K 14/705

(54) **Cellular antanonist**

(30) Priority: 09.06.2006 GB 0611451; 27.06.2006 US 816971 P
(71) Applicant: Institut Pasteur Korea, Seongbuk-gu Seoul 136-791 (KR)
(72) Inventor: Emans, Neil, Seoul, South Korea 136-791 (KR); Altschuler, Yoram c/o Institut Pasteur Korea, Seol, South Korea 136-791 (KR); Kwon, Yongjun, Kangwon-do, South Korea 220-130 (KR)
(74) Representative: Engelhard, Markus

(57) **Abstract**

A method for identifying a compound that modifies the cellular distribution of a GPCR in a population of cells comprising:
a) taking a population of cells expressing a GPCR
b) incubating said cells with a candidate compound and with a ligand of the GPCR
c) determining distribution of said GPCR in cells treated according to step b) and comparing with distribution of GPCR in cells incubated with a ligand of the GPCR in the absence of the candidate compound;

wherein altered distribution in cells incubated with the candidate compound is indicative that the candidate compound modifies GPCR cellular distribution.

## Description

### SUMMARY OF THE INVENTION

The present invention provides a new approach for the treatment of G-protein coupled receptor based disease. This approach comprises altering the physiology of G-protein coupled receptor based responses by exerting chemical control over the cellular distribution of the receptor. This approach allows a method for phenotypic screening for candidate compounds which disrupt GPCR distribution signalling activity and are therefore candidates for the treatment of GPCR-related conditions. Compounds that create an altered distribution of GPCR upon exposure to the cell are identified. The method then uses these molecules to alter physiology through their effect on receptor distribution and reduction to practice is demonstrated in an ex-vivo model of g-protein coupled receptor activity.

### BACKGROUND OF THE INVENTION

Signal transduction from cell surface G-protein coupled receptors (GPCRs) is fundamental to the coordination of intracellular responses to alterations in the extra-cellular concentration of growth factors, hormones, and neurochemicals. This signaling is of critical importance for physiological homeostasis in systems as diverse as blood pressure regulation, odor recognition, and visual perception ¹. More than half of the available prescription drugs target G-protein coupled receptors and most can be described as agonists or antagonists of G protein coupled receptor activity. Generally, drug design efforts are aimed at the manipulation of the interaction(s) between a receptor and its endogenous ligands, whether to repress this through chemical antagonism or stimulate receptor activity through the use of artificial agonists.

G-protein coupled receptors are transmembrane proteins with a ligand-binding site exposed on the outside surface of the cell and an effector site that extends into the cytosol. Upon ligand (agonist) binding, conformational changes in heptahelical receptors recruit specific heterotrimeric G-proteins ², enable their dissociation into Gα and Gβγ subunits and results in the activation of the Gα subunit. This, in turn, activates a series of class specific downstream effectors, such as phospholipase C-β for the Gq class and adenyl cyclase for Gs, which then produce changes in the levels of second messengers such as intracellular Ca++ and cyclic AMP (CAMP) ³.

Agonist induced internalization is a property of many GPCRs, such as the β-adrenergic, endothelin A, and µ opioid receptors ⁴. Internalization controls signaling for it desensitizes cellular responses as receptors are removed from the cell surface. It is also implicated in hypertension and drug tolerance. It demonstrates the importance of cellular location in receptor function.

Activated receptors at the cell surface are rapidly desensitized from G proteins through phosphorylation by G-protein receptor kinases (GRKs) ⁵ and the subsequent recruitment of the arrestin adaptor proteins ^{6,7}. Arrestins promote the internalization of the receptor into plasma membrane derived vesicles, either caveolae or clathrin-coated vesicles, which pinch off into the cell interior to deliver the receptor into the endosomal organelles. Once internalized, GPCRs have several endocytic itineraries and may be transported for degradation, rapidly recycled to the cell surface, or resensitize and recycle from the pericentriolar recycling compartment to the cell surface and enable another cycle of signaling.

Blood pressure is under the control of the coordinated action of biochemical stimuli that mediate vascular smooth muscle tone, and a prime stimulus are the endothelin hormones..

Endothelins are the most potent vasoconstrictors yet identified and are implicated in blood pressure regulation, cardiac development and cancer ⁸⁻¹². These hormones exert their physiological effect through the activation of the endothelin A and endothelin B GPCRs, which differ in terms of their G-protein coupling and trafficking. Agonist binding to the endothelin A receptor acts via Gq signaling to promote vascular constriction through second messengers. The endothelin A receptor (ETAR) undergoes agonist induced internalization and mediates vasoconstriction via activation of both Gq and Gs. In contrast, the endothelin B receptor (ETBR) mediates vasodilation and is constitutively internalized ¹³⁻¹⁵. Clinically, medications targeted at the endothelin system antagonize agonist activity and disrupt endothelin: receptor interactions (bosentan: ¹⁶).

Current pharmaceutical approaches to modulating GPCRs are aimed at the interaction of the receptor with its agonist, enhancing receptor activation through the addition of chemical agonists or disrupting the receptor: agonist interaction through the administration of chemical antagonists.

However, standard agonists and antagonists that target GPCRs have a number of disadvantages. For example, they must be administered in high doses in order to reach the required concentration. In addition, higher doses are required as patients develop drug resistance especially where they are administered long term for the treatment of chronic pain and blood pressure control, etc.

Treating GPCR related diseases remains a challenge, and this has been approached through the introduction of drugs acting as agonists and antagonists. More than a quarter of the leading 100 drugs on the market are GPCR related and have a combined value of more than 30 billion dollars annually. In a broader sense, receptors serve as the drug target for approximately half of known drugs. Given the importance of this set of pharmaceutical targets, new strategies for drug identification may provide new drugs for GPCR diseases.

Accordingly, there is a need for alternative strategies for developing drugs which treat GPCR-related conditions.

### SUMMARY OF THE INVENTION

The present invention provides a new approach for the treatment of G-protein coupled receptor based disease. This approach comprises altering the physiology of G-protein coupled receptor based responses by exerting chemical control over the cellular distribution of the receptor. This forms the basis for a method of phenotypic screening for candidate compounds which disrupt GPCR distribution signalling activity and are therefore candidates for the treatment of GPCR-related conditions. Compounds that create an altered distribution of GPCR upon exposure to the cell are identified. The ability of these molecules to alter physiology through their effect on receptor distribution is demonstrated in an ex-vivo model of G-protein coupled receptor activity.

This approach is referred to a "cellular antagonism" and the candidate compounds used within it are referred to as "cellular antagonists" with "cellular antagonism" being the intervention in the function of a GPCR by modulating its location or signalling properties within the cell. Advantageously, such cellular antagonists are required in much lower concentrations to modify GPCR function compared to conventional GPCR modulating compounds.

Accordingly in a first aspect the present invention provides a method for identifying a compound that modifies the cellular distribution of a GPCR in a population of cells comprising:
a) taking a population of cells expressing a GPCR
b) incubating said cells with a candidate compound and with a ligand of the GPCR
c) determining distribution of said GPCR in cells treated according to step b) and comparing with distribution of GPCR in cells incubated with a ligand of the GPCR in the absence of the candidate compound;
wherein altered distribution in cells incubated with the candidate compound is indicative that the candidate compound modifies GPCR cellular distribution.

G-protein coupled receptors (GPCRs) are characterised as 7 transmembrane receptors. Suitably, the GPCR for use in the method of the present invention is one that undergoes agonist-induced receptor internalization. Such GPCRs includes muscarinic receptors among others. In one embodiment, the GPCR is a β-adrenergic, endothelin A , kappa opioid receptor or µ opioid receptor. In accordance with the present invention, the term GPCR includes both naturally occuring GPCRs as well as GPCRs that have been modified.

GPCRs are generally found at the cell surface. However, following receptor activation through ligand binding, many receptors are internalised and then recycled to the cell surface through the intracellular endosome pathway or routed for transcellular transport or destruction in degradative lysosomal compartments. Accordingly, in the method of the present invention, "altered distribution" or "altered cellular distribution" of GPCR in cells is measured by reference to cellular distribution of GPCR, following receptor activation, in a control assay cell which has not been treated with a candidate compound.

In one embodiment, GPCR location can be detected through antibody-based recognition of GPCR and visualisation of an antibody labelled GPCR. In another embodiment, the GPCR may also express a detectable tag.

In particular, the cellular distribution of GPCR (i.e. whether it is located at the cell surface or at an intracellular location) may be determined by detecting a labelled GPCR in treated and untreated cells. By "labelled GPCR" is meant a GPCR which has been modify to comprise a detectable label or marker. Suitable labels and markers for use in such protein location studies will be familiar to those skilled in the art and include labels and markers as described herein.

In one embodiment, distribution of the GPCR is determined by microscopy. Suitable microscopic methods include optical microscopy, confocal microscopy or automated microscopic screening methods. Optical microscopy is well suited to the study of GPCR activation and trafficking, and automated optical screening of cell based GPCR activation assays (high content screening^{19,20}) provides the means to analyze these pathways on a larger scale than previously possible²¹. Additionally, automated microscopic screening enables the tracing of endocytosis of activated receptors and currently serves for the screening of novel drugs.

An indication that a candidate compound is one that modifies GPCR cellular distribution is given by an altered cellular distribution of GPCR in a cell treated with a candidate compound when compared to a cell which has not been treated with a candidate compound. Methods for obtaining data images of treated and control cells and performing a comparison of distribution are described herein. In particular, a comparison may be made between levels of labelled GPCR at the cell surface, in the early endosomes or in the recycling endosomes. In one embodiment, the distribution of GPCR is compared by measuring labelled GPCR at the cell surface, or in peripheral endosomes or the peri-centriolar recycling endosome. In another embodiment of the invention, a candidate compound that modifies the cellular distribution of a GPCR is one that gives relative numbers of labelled endosomes outside a threshold of +/two to three times the standard deviation of the untreated but ligand treated control.

Advantageously, since the candidate compound molecules are identified by the phenotype of ability to alter cellular distribution of the GPCR, and then confirmed by physiological testing, a definition of the 'target' of the molecules is not required.

In order to identify a candidate compound as one that is specific for GPCR cellular distribution and or one which has an overall effect on the cellular endocytosis pathway, an assay to determine the effect of a candidate compound on house keeping endocytosis can be performed. "House keeping endocytosis" is the normal process by which cell surface proteins are recycled. Accordingly, in one embodiment, the method of the present invention further comprises comparing the effect of the candidate compound on the distribution of said labelled GPCR with its effect on house keeping endocytosis.

Suitably "house keeping endocytosis" can be measured by measuring internalisation of a marker.

Accordingly, in one embodiment of the invention there is provided a method in accordance with the invention wherein the effect on "house keeping endocytosis" is determined in a method comprising:
a) incubating cells with a marker
b) incubating cells with a candidate compound
c) determining the distribution of the marker in cells incubated with a candidate compound and comparing with distribution of marker in cells not incubated with a candidate compound wherein altered location in cells incubated with a candidate compound is indicative of a compound that modifies "house keeping endocytosis".

Suitably the marker for internalisation is a labelled compound such as labelled dextran. In one embodiment, the labelled dextran is a BODIPY™-labelled dextran such as the labelled dextran described herein.

In another embodiment of the invention, the cells which are used for the assay in accordance with the invention and those for measuring house keeping endocytosis are the same. In this embodiment, the determination of GPCR location and marker internalisation are carried out simultaneously. In this embodiment the label on the GPCR and the label on the marker are suitably detectably different. Accordingly, there is provided a method in accordance with the invention wherein the determination of the effect of the candidate compound on GPCR distribution and on labelled dextran distribution is determined simultaneously.

GPCR ligands are those compounds which bind to and activate a GPCR. Suitable ligands for a particular GPCR will be known to those skilled in the art and include agonists of GPCRs. For example, where the GPCR is endothelin A receptor, suitable ligands include endothelin-1.

Suitably, in the method of the invention, an increase in GPCR at the cell surface is indicative of a candidate compound for treating a GPCR related disease such as a disease related to aortal superconstriction. Furthermore, an increase in intracellular GPCR is indicative of a candidate compound for slowed aortal vasoconstriction.

In a further embodiment of the invention, candidate compounds which have been selected according to the method of the invention can be used in additional assays to confirm their activity. Suitably, a method of the invention further comprises taking the candidate compound identified from steps a) to c) in accordance with the first aspect of the invention and determining its effect in an assay for vasoconstriction. Suitable assays for vasoconstriction include an ex-vivo rat thoracic aorta constriction-relaxation model.

In another aspect of the invention there is provided a method of modulating GPCR distribution in a cell comprising administering an inhibitor of PKC or PKA.

Candidate compounds identified in accordance the present invention are candidates for use in the treatment of GPCR related diseases. A large number of GPCR related diseases are familiar to those skilled in the art and suitable diseases are described herein.

Accordingly, in another aspect, the invention provides a method of treating a GPCR related disease comprising administering a compound that modifies the cellular distribution of a GPCR.

In a further aspect there is provided a compound that modifies cellular distribution of a GPCR for use in the treatment of a GPCR related disease.

Suitable compounds have been identified herein and include the protein kinase C inhibitors RO 31 8220, Gö 6976, palmitoyl DL carnitine chloride, protein kinase A inhibitors KT 5720, H-89 and erbstatin A.

In another aspect, the invention provides a use of a compound that modifies the cellular distribution of a GPCR in the preparation of a medicament for use in the treatment of a GPCR related disease. Suitably, the compound is selected from the protein kinase C inhibitors RO 31 8220, Gö 6976, palmitoyl DL carnitine chloride, protein kinase A inhibitors KT 5720, H-89 and erbstatin A.

In one embodiment of any of these aspects, the GPCR related disease may be endothelin related pulmonary arterial hypertension. Suitable the GPCR related disease is related to impaired vascular tone and can be examined in the rat thoracic aorta vasoconstriction model.

### BRIEF DESCRIPTION OF THE FIGURES

### Figure Legends

### Figure 1

High content screen of endothelin A receptor and housekeeping endocytosis against a library of kinase inhibitors.

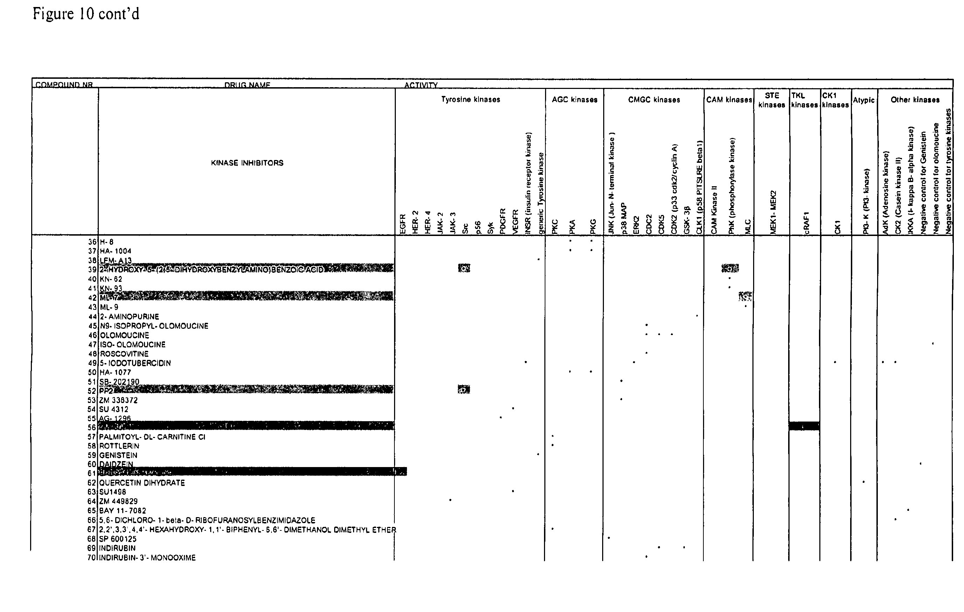

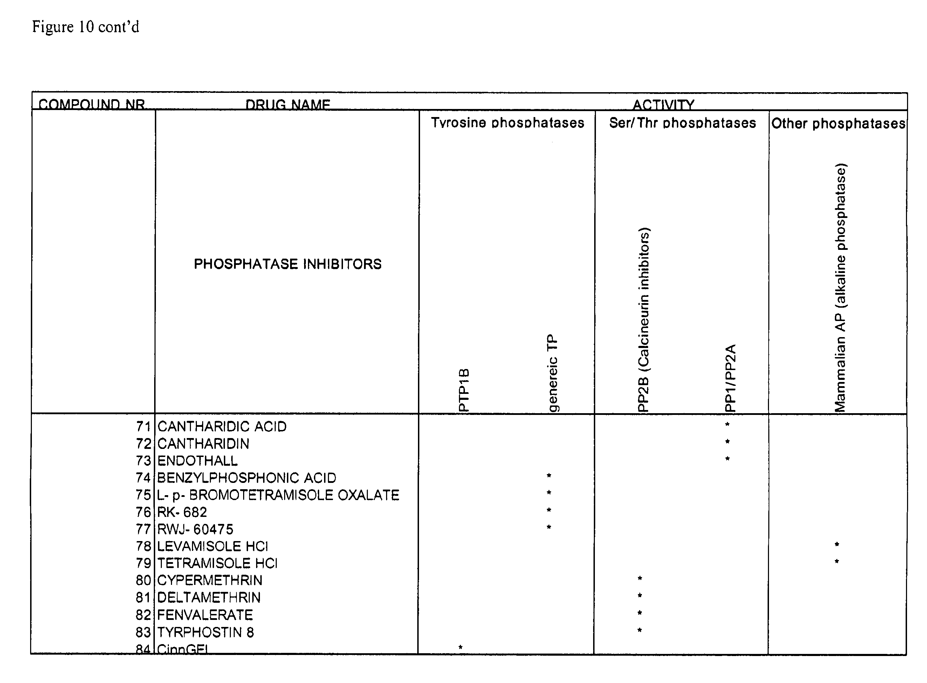

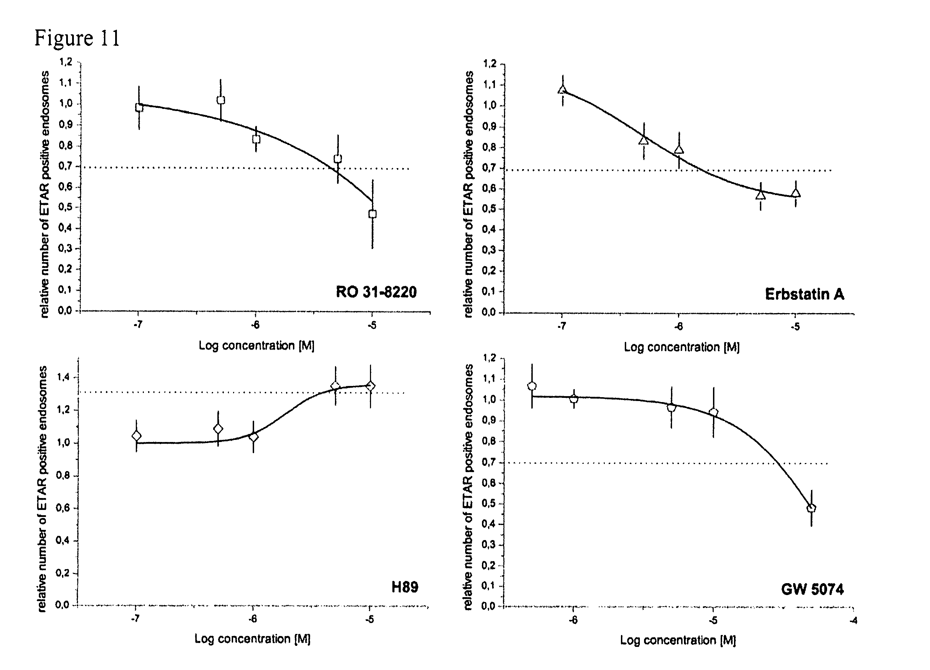

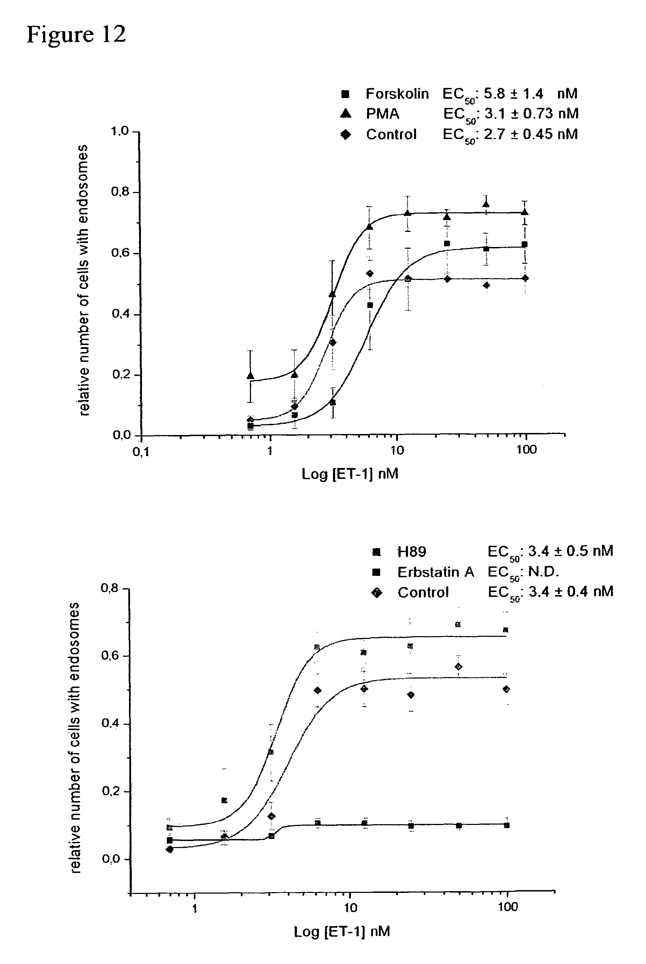

Quantitation of Endothelin induced internalization of the endothelin A receptor eGFP fusion into recycling endosomes. (**a**) The endothelin A receptor GFP fusion protein is localized to the plasma membrane in stably transfected HEK293 cells. (**b**) The endothelin A receptor GFP fusion is internalized into a pericentriolar endosome upon stimulation with 40 nM endothelin-1. (**c**) Endocytic uptake of a green fluorescent fluid phase dextran is blocked at 4°C and (**d**) is robust in cells at 37°C counter stained with red fluorescent cell stain Syto 60.(**e**) The dose-response curve for endothelin on internalization of the receptor derived with automated image analysis. The relative number of cells showing internalization is plotted against endothelin concentration. (**f**) The relative number of endosomes for the ETAR assay (●) and the housekeeping endocytosis assay (■) is plotted against the compounds. The black dotted line indicates the average value of the positive control that was used to normalize the data for both assays. C- ETAR and C- HK are the relative values for the negative controls for the ETAR assay (●) and the housekeeping assay (■), respectively. Hits are defined as falling outside ± 3 standard deviations from the mean for the ETAR (---) or housekeeping assay (---). The red lines connecting the relative values of the two complementary assays indicate the specificity of the compound for the ETAR or the housekeeping assay. The greater this line grows the greater the difference between the assay results and the higher the specificity of the compound. The relative data for the following compounds are labeled: 6: staurosporine, 20:Tyrphostin-9, 28: AG-879, 31: GF 109203X, 32: Hypericine, 33: Ro 31-8220, 35: H-89, 61: Erbstatin Analog, 65: BAY 11-7082. Results are the mean of 2-4 individual experiments, where each point comprises 5 replicate wells and 5 analyzed image pairs per well with an n (cells) > 1500 per experimental point. (**g**) Rotary map of the kinase inhibitor library targets where arc corresponds to number of compounds within each subfamily and depth to the diversity of each inhibitors' family. A complete list of the compounds is provided in Figure 10.

### Figure 2

Differential effects of compounds on ETAR trafficking and housekeeping endocytosis. Confocal image pairs are of endothelin-1 stimulated ETAR-GFP cells (upper panels) or fluorescent dextran internalization (lower panels). All compounds were used at 10µM. Scale bar 20 µm.

### Figure 3

Single cell differential screening. Endothelin A receptor internalization was imaged after stimulation with 40 nM Endothelin and a 10 min internalization of red fluorescent dextran on a confocal microscope in presence of the following compounds: (**a**) 10 µM Ro 31-2880. (**b**) 10 µM Erbstatin A. (**c**) 10 µM H-89 (**d**) DMSO (e) 40 nM endothelin-1 alone. Confocal images,are from a 1 µm Z section. The endothelin A receptor is shown in green, the dextran in red. Scale bar 5 µm.

### Figure 4

Sensitivity of GPCR internalization to kinase inhibitors acting on ETAR endocytosis. (**a**) Fully polarized MDCK cells expressing the M1AR were treated with 100nM carbachol, DMSO, or compounds then fixed and stained with antibodies against the M1AR epitope tag (green) or the GP130 apical plasma marker (red). Cells were pretreated with compounds : 10 µM RO 31 8220, 10µM Erbstatin A, 10µM H-89, with 100 nM agonist, agonist alone or DMSO. Scale bar: 20µm. (**b**) HEK293T cells expressing a human kappa Opioid receptor fusion to the green fluorescent protein were treated with 10 µM Gö 6976, 10µM Erbstatin A, 10µM H-89, 10 µM KT-5720 with 300 nM agonist, agonist alone or DMSO. Scale bar 20 µm. (**c**) Quantification of kappa Opioid receptor internalization (endosomes/cell) after compound treatment and agonist treatment.

### Figure 5

Effect of protein kinase C inhibition and stimulation on endothelin A receptor internalization. (**a**) ETAR internalization and housekeeping endocytosis were measured against a panel of protein kinase C inhibitors. All inhibitors were used at 10µM, (**b**) ETAR internalization was measured after 100nM Phorbol ester, 10µM RO 31 8220 or both, and (**c**) Gö 6976. (**d**) ETAR cells imaged under identical conditions after 24 hour exposure to 0 ,1 ,10, 100 nM phorbol ester (**e)** Quantitation of cell surface intensity using the absolute image gradient (**e**) endothelin induced endothelin receptor internalization after 24 exposure to 0 ,1 ,10, 100 nM phorbol ester, washout and stimulation with endothelin-1 in the presence of 0,10 or 100 nM Phorbol ester (*** p<0.002 compared to control). Scale bar 20µm.

### Figure 6

Effect of protein kinase A inhibition on endothelin A receptor internalization. ETAR and housekeeping internalization were measured for a set of PKA inhibitors (**a**), and ETAR internalization was measured after (**b**) KT5720, H89 or forskolin and (**c**) the number of endosomes /cell was quantified after agonist stimulation.

### Figure 7

Effect of tyrosine kinase A inhibition on endothelin A receptor internalization. ETAR and housekeeping internalization were measured for a set of tyrosine inhibitors (**a**), and ETAR internalization was measured after (**b**) single and combinatorial treatment with 100nM phorbol ester, RO 31 8220, erbstatin A and H-89.

### Figure 8

cAMP production requires sequential G-protein activation. Cells were treated with 10µM RO 31 8220, H-89, KT 5720 Forskolin or Erbstatin A, then stimulated with 40nM ET-1 and cAMP was measured over time by indirect ELISA using standard concentration cAMP curves.

### Figure 9

Cellular antagonism of endothelin receptor action directly affects aortal contraction ex-vivo. Representative aortal constriction data for (**a**) 40nM endothelin 1 alone or with 10µM Gö6976, or 10µM Erbstatin A, or 10µM H-89 pre-treatment prior to and during agonist addition. Data were normalized to the noradrenaline induced contraction prior and the maximal extent for contraction is plotted as closed circles (**b**) Mean maximal aortal strip constriction after compound treatment as above for 12 aortal strips (3 experiments per compound).

### Figure 10

Summary of the compounds screened and the target enzymes (*), compounds affecting the ETAR GPCR assay are indicated in black, lethal effects by grey.

### Figure 11

Secondary screening of the kinase inhibitor effect on ETAR endocytosis. Endothelin receptor internalization was measured after treatment with a range of compound concentrations from 0.1 to 50 µM. Relative internalization of the ETAR is plotted against compound concentration for Ro 31-8220, the erbstatin Analogue, H-89 and GW 5074. Results are the mean of four experiments, showing the standard deviation, where each experimental point comprises five pairs of images giving an n (cell) of > 4000 per compound concentration. Dotted lines indicate the ± 3 SD threshold for receptor internalization in control cells.

### Figure 12

EC50 titrations were performed in the presence of 10 µM of the kinase inhibitors, with the fitted EC50 values displayed in nM. Results are the mean of four experiments, showing the standard deviation, where each experimental point comprises five pairs of images giving an n (cell) of > 4000 per compound concentration.

### DETAILED DESCRIPTION OF THE INVENTION

Unless defined otherwise, all technical and scientific terms used herein have the same meaning as commonly understood by one of ordinary skill in the art (e.g., in cell culture, molecular genetics, nucleic acid chemistry, hybridisation techniques and biochemistry). Standard techniques are used for molecular, genetic and biochemical methods. See, generally, Sambrook et al., Molecular Cloning: A Laboratory Manual, 2d ed. (1989) Cold Spring Harbor Laboratory Press, Cold Spring Harbor, N.Y. and Ausubel et al., Short Protocols in Molecular Biology (1999) 4th Ed, John Wiley & Sons, Inc.; as well as Guthrie et al., Guide to Yeast Genetics and Molecular Biology, Methods in Enzymology, Vol. 194, Academic Press, Inc., (1991), PCR Protocols: A Guide to Methods and Applications (Innis, et al. 1990. Academic Press, San Diego, Calif.), McPherson et al., PCR Volume 1, Oxford University Press, (1991), Culture of Animal Cells: A Manual of Basic Technique, 2nd Ed. (R. I. Freshney. 1987. Liss, Inc. New York, N.Y.), and Gene Transfer and Expression Protocols, pp. 109-128, ed. E. J. Murray, The Humana Press Inc., Clifton, N.J.). These documents are incorporated herein by reference.

As used herein, the term "GPCR-related disease" includes any disease or disorder associated with aberrant GPCR signaling, including, but not limited to, neuropsychiatric disorders such as, for example, schizophrenia, bipolar disorders and depression; cardiopulmonary disorders such as, for example, cardiachypertrophy, hypertension, thrombosis and arrhythmia; inflammation, cystic fibrosis and ocular disorders. Without limitation as to mechanism, GPCR-related diseases are generally associated with decreased GPCR-signaling.

As used herein, the term "GPCR ligand" and "GPCR agonist" includes any molecule or agent which binds to a GPCR and elicits a response. As used herein, the term "GPCR antagonist" includes any molecule or agent which binds to a GPCR but which does not elicit a response.

'Cellular antagonism' is defined as intervening in the function of a GPCR by modulating its location, or signaling properties within a cell using a small molecule modulator (compound). In the simplest conception, cellular antagonism acts at the level of the receptor.

As used herein, the term "altered" cellular distribution includes an alteration in a detectable distribution compared to a distribution in a control cell.

"Candidate compound" means any solution, compound, or other substance (including, but not limited to, small molecules such as deoxyribonucleotide and ribonucleotide molecules as well as peptides, proteins, and nucleic acids) to be screened according to the methods described herein for altered GPCR cellular distribution.

The invention provides methods (also referred to herein as "screening assays") for identifying candidate or test compounds or agents comprising therapeutic moieties (e. g. peptides, peptidomimetics, peptoids, polynucleotides, small molecules or other drugs) which alter the cellular distribution of a GPCR.

The test compounds of the present invention are generally either small molecules or bioactive agents. In one preferred embodiment, the test compound is a small molecule. In another preferred embodiment, the test compound is a bioactive agent. Bioactive agents include, but are not limited to, naturally-occurring or synthetic compounds or molecules ("biomolecules") having bioactivity in mammals, as well as proteins, peptides, oligopeptides, polysaccharides, nucleotides and polynucleotides.

The candidate compounds of the present invention may be obtained from any available source, including systematic libraries of natural and/or synthetic compounds.

The candidate compound to be tested may be administered to the cell in several ways. For example, it may be added directly to the cell culture medium or injected into the cell. Alternatively, in the case of polypeptide agents, the cell may be transfected with a nucleic acid construct, which directs expression of the polypeptide in the cell. Preferably, the expression of the polypeptide is under the control of an inducible promoter.

Cells useful for assays and methods in accordance with the present invention include eukaryotic and prokaryotic cells, including, but not limited to, bacterial cells, yeast cells, fungal cells, insect cells, nematode cells, plant cells, and animal cells. Suitable animal cells include, but are not limited to, HEK cells, HeLa cells, COS cells, U20S cells,CHO-K1 cells, and various primary mammalian cells.

Cells useful in the present invention may stably or transiently express the labelled GPCRs used in the methods described herein. Methods of expressing genes using non-mammalian viruses (e. g. , baculoviruses) described in U. S. Patent Nos. 4,745, 051; 4,879, 236; 5,348, 886; 5,731, 182; 5,871, 986; 6,281, 009; and 6,238, 914; may be used in the present methods.

Labels or marker molecules that may be used to conjugate with the GPCR include, but are not limited to, molecules that are detectable by spectroscopic, photochemical, radioactivity, biochemical, immunochemical, colorimetric, electrical, or optical means, including, but not limited to, bioluminescence, phosphorescence, and fluorescence. These labels should be biologically compatible molecules and should not compromise the ability of the GPCR to interact with its ligand or with the GPCR signalling system or receptor internalisation system, and the interaction of the ligand with the GPCR must not compromise the ability of the label to be detected.

Labels include radioisotopes, epitope tags, affinity labels, enzymes, fluorescent groups, chemiluminescent groups, and the like. Labels include molecules that are directly or indirectly detected as a function of their interaction with other molecule (s) as well as molecules detected as a function of their location or translocation. In some embodiments, the labels are optically detectable marker molecules, including optically detectable proteins, such that they may be excited chemically, mechanically, electrically, or radioactively to emit fluorescence, phosphorescence, or bioluminescence. Optically detectable marker molecules include, for example, beta-galactosidase, firefly luciferase, bacterial luciferase, fluorescein, Texas Red, horseradish peroxidase, alkaline phosphatase, and rhodamine-conjugated antibody.

In other embodiments, the optically detectable labels or marker molecules are inherently fluorescent molecules, such as fluorescent proteins, including, for example, Green Fluorescent Protein (GFP).

The label may be conjugated to the GPCR by methods as described in U. S. Patent Nos. 5,891, 646 and 6,110, 693. The label may be conjugated to the GPCR at the front-end, at the back-end, or in the middle. In some embodiments, the labels are molecules that are capable of being synthesized in the cell. The cell can be transfected with DNA so that the conjugate of label and a GPCR is produced within the cell. As one skilled in the art readily would understand, cells may be genetically engineered to express the conjugate of GPCR and a label by molecular biological techniques standard in the genetic engineering art. Suitable methods are described herein with particular reference to GFP tagged GPCRs.

Methods of detecting the intracellular location or concentration labelled GPCR will vary depending upon the label used. One skilled in the art readily will be able to devise detection methods suitable for the label used. For optically detectable labels, any optical method may be used where a change in the fluorescence, bioluminescence, or phosphorescence may be measured due to a redistribution orreorientation of emitted light. Such methods include, for example, polarization microscopy, bioluminescence resonance energy transfer (BRET), fluorescence resonance energy transfer (FRET), evanescent wave excitation microscopy, and standard or confocal microscopy.

### EXPRESSION

The term "expression" refers to the transcription of a genes DNA template to produce the corresponding mRNA and translation of this mRNA to produce the corresponding gene product (i.e., a peptide, polypeptide, or protein).

### AGONISTS AND ANTAGONISTS

Agents capable of activating or increasing the signalling from a GPCR are referred to as agonists.

Agents capable of reducing, inhibiting or blocking the activity of a GPCR are referred to as antagonists.

### PHARMACEUTICALS

The agents that alter the cellular distribution of a GPCR will typically be formulated into a pharmaceutical composition. In this regard, and in particular for human therapy, even though the agents described herein can be administered alone, they will generally be administered in admixture with a pharmaceutical carrier, excipient or diluent selected with regard to the intended route of administration and standard pharmaceutical practice.

By way of example, in the pharmaceutical compositions, the agents may be admixed with any suitable binder(s), lubricant(s), suspending agent(s), coating agent(s), or solubilising agent(s).

Tablets or capsules of the agents may be administered singly or two or more at a time, as appropriate. It is also possible to administer the agents in sustained release formulations.

Thus, the present invention also provides a method of treating GPCR related disease in a subject comprising administering to said subject an effective amount of a candidate compound identified in accordance with the invention.

Typically, the pharmaceutical compositions - which may be for human or animal usage - will comprise any one or more of a pharmaceutically acceptable diluent, carrier, excipient or adjuvant. The choice of pharmaceutical carrier, excipient or diluent can be selected with regard to the intended route of administration and standard pharmaceutical practice. As indicated above, the pharmaceutical compositions may comprise as - or in addition to - the carrier, excipient or diluent any suitable binder(s), lubricant(s), suspending agent(s), coating agent(s), solubilising agent(s).

It will be appreciated by those skilled in the art that the agent may be derived from a prodrug. Examples of prodrugs include certain protected group(s) which may not possess pharmacological activity as such, but may, in certain instances, be administered (such as orally or parenterally) and thereafter metabolised in the body to form an agent that is pharmacologically active.

The agent may be administered as a pharmaceutically acceptable salt. Typically, a pharmaceutically acceptable salt may be readily prepared by using a desired acid or base, as appropriate. The salt may precipitate from solution and be collected by filtration or may be recovered by evaporation of the solvent.

### ADMINISTRATION

The term "administered" includes delivery by viral or non-viral techniques. Viral delivery mechanisms include but are not limited to adenoviral vectors, adeno-associated viral (AAV) vectos, herpes viral vectors, retroviral vectors, lentiviral vectors, and baculoviral vectors. Non-viral delivery mechanisms include lipid mediated transfection, liposomes, immunoliposomes, lipofectin, cationic facial amphiphiles (CFAs) and combinations thereof.

The components may be administered alone but will generally be administered as a pharmaceutical composition - e.g. when the components are in admixture with a suitable pharmaceutical excipient, diluent or carrier selected with regard to the intended route of administration and standard pharmaceutical practice.

For example, the components can be administered in the form of tablets, capsules, ovules, elixirs, solutions or suspensions, which may contain flavouring or colouring agents, for immediate-, delayed-, modified-, sustained-, pulsed- or controlled-release applications.

If the pharmaceutical is a tablet, then the tablet may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavouring agents, colouring matter or dyes, with emulsifying and/or suspending agents and with diluents such as water, ethanol, propylene glycol and glycerin, and combinations thereof.

The routes for administration (delivery) may include, but are not limited to, one or more of oral (e.g. as a tablet, capsule, or as an ingestable solution), topical, mucosal (e.g. as a nasal spray or aerosol for inhalation), nasal, parenteral (e.g. by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, vaginal, epidural, sublingual.

Conveniently, administration may be by inhalation. Commercially available nebulisers for liquid formulations, including jet nebulisers and ultrasonic nebulisers are useful for such administration. Liquid formulations can be directly nebulised and lyophilised powder can be nebulised after reconstitution.

For administration by inhalation, the agents are conveniently delivered in the form of an aerosol spray presentation from pressurised packs or nebulisers. The agents may also be delivered as powders which may be formulated and the powder composition may be inhaled with the aid of an insufflation powder inhaler device.

### DOSE LEVELS

Typically, a physician will determine the actual dosage which will be most suitable for an individual subject. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

### FORMULATION

The component(s) may be formulated into a pharmaceutical composition, such as by mixing with one or more of a suitable carrier, diluent or excipient, by using techniques that are known in the art.

### KITS

The materials for use in the present invention are ideally suited for the preparation of kits.

Such a kit may comprise containers, each with one or more of the various reagents (optionally in concentrated form) utilised in the methods, including, a cell that expresses or is capable of expressing a labelled GPCR. The kit optionally further comprises one or more controls. A set of instructions will also typically be included.

The invention will now be further described by way of Examples, which are meant to serve to assist one of ordinary skill in the art in carrying out the invention and are not intended in any way to limit the scope of the invention.

### EXAMPLES

### Materials and Methods

### Chemicals

All fine chemicals were purchased from Sigma-Aldrich. Fluorophores and their reactive forms were purchased from Molecular Probes (Eugene, USA). DRAQ5 was from BioStatus (Shepshed, UK). 40 kDa BODIPY-Fl-dextran was synthesized from amino dextran using standard protocols. The lyophilized product had a typical labeling ratio of 3 fluorophores·mol⁻¹. Kinase and phosphatase inhibitors were purchased as 95-99% pure 10 mM stock solutions in dimethylsulfoxide or water (Biomol Hamburg, Germany). Stock solutions and formatted assay plates were stored at -20°C. CAMP was measured by indirect ELISA (R & D systems, MN USA) following the manufacturers protocol for CAMP acetylation, and a CAMP standard.

### Cell lines and cell culture

HeLa cells were obtained from the German Collection of Microorganisms and Cell Cultures (Braunschweig, Germany). HeLa cells were cultivated in phenol red free Dulbecco's modified eagles medium (Invitrogen; Carlsbad, USA) supplemented with 10% foetal calf serum (Biochrom; Berlin, Germany) and 1% penicillin streptomycin (Invitrogen; Carlsbad, USA). HEK 293 cells were cultivated in Dulbecco's modified eagle's medium/F12 (Invitrogen; Carlsbad, USA) supplemented with 10% foetal calf serum, 1% penicillin streptomycin and 1% genetecin.

A human endothelin A receptor cDNA clone in pCDNA3.1(-) (Invitrogen Carlsbad, USA) was excised via *Kpn*I/*Hind*III and fused to the *egfp* coding sequence of the pEGFP-N1 vector (Clontech, Palo Alto USA) by splice overlap PCR using specific primers. The amplified fragment was digested via *Kpn*I/*Eco*RV and ligated in the pCDNA3.1(-) vector digested with the same combination of restriction enzymes. The resulting pETAR-EGFP DNA was cloned, checked by sequencing and used for transfection of HEK 293 cells using standard protocols. A recombinant clone was obtained through several cycles of selection and limiting dilutions. For screening, cells were passed onto coverslip bottomed 96 well plates (Greiner, Longwood, USA) at a density of 4·10³ cells/well for Hela and 5·10³ cells/well for HEK293 cells, 48 hours in advance. A human kappa opioid receptor cDNA clone in pcDNA3.1(+) (invitrogen) was amplified by splice overlap PCR using specific primers. The amplified fragment was digested via *Nhe*I/ *KpnI* and ligated in the pEGFP-N3 vector (Clontech) digested with the same combination of restriction enzymes. The resulting pKappa-EGFP DNA was cloned, checked by sequencing and used for transfection of HEK 293 cells using standard protocols.

### Cell imaging

The endothelin receptor translocation assay and the housekeeping endocytosis assay were screened on the Opera ultra-High throughput confocal screening system (Evotec Technologies, Hamburg; Germany). The Opera is a fully automated 4 color laser excitation confocal system (405, 488, 532, 637 nm) based on an inverted microscope architecture to image cells cultivated in coverslip bottomed microtitre plates. Images were acquired with 0.7 NA 20x water immersion or 40x 0.X NA water immersion lenses (Olympus instruments, Japan) at room temperature with confocality generated by a nipkow disc system and image acquisition with 3 parallel integrated 16 bit CCD cameras. Images were corrected for optical vignetting using the Opera acquisition software. Image analysis used custom written scripts within the Opera software. Images were exported as 16 bit *.TIFF files and scaled with Metamorph (Universal Imaging, West Chester PA) before export to Adobe Photoshop. Coverslip grown cells were imaged using a Leica TCS SP confocal with a 40 x oil objective using 488 nm and 568 nm excitation and 510-530 and 590-620 nm emission filter settings, respectively. Images were uniformly scaled, processed and overlaid using Metamorph (Universal Imaging, West Chester PA).

### Cell based screening

Compounds in DMSO or H₂O were diluted into 1% serum (HEK screens) or serum free (Hela screens) buffered media at the working concentration (0.1 nM to 10 µM) just prior to screening. For the GPCR internalization assay, recombinant HEK cells were plated in 96 well plates and incubated overnight in DMEM/F12 containing 1% FBS. The plates were washed and the cells incubated for 120 min under tissue culture conditions with the compounds in 1% serum medium. The compound solutions were then supplemented with 40 nM endothelin-1 and 10 µM Syto60. Cells were incubated for 120 min at 22°C. Plates were imaged by the Opera using 488/633 nm excitation and 510 nm (50 nm bandpass) or 680 (50 nm bandpass) filters in parrallel. Typically, 5 image pairs were acquired per well.

For the housekeeping assay, HeLa cells in 96 well plates were washed and incubated for 120 min with the compounds in serum free buffered medium under tissue culture conditions. The medium was then replaced with serum-free buffered medium supplemented with 1 mg/ml BODIPY-FL-Dextran and 10 µM Syto60. Cells were incubated for 20 min at 37°C, then placed on a cooled block and washed extensively with ice cold phosphate buffered saline 1 % w/v bovine serum albumin (Serva; Heidelberg, Germany). Plates were imaged by the Opera using 488/633 nm excitation and 510 nm (50 nm bandpass) or 680 (50 nm bandpass) filters.

### Cell based assay evaluation

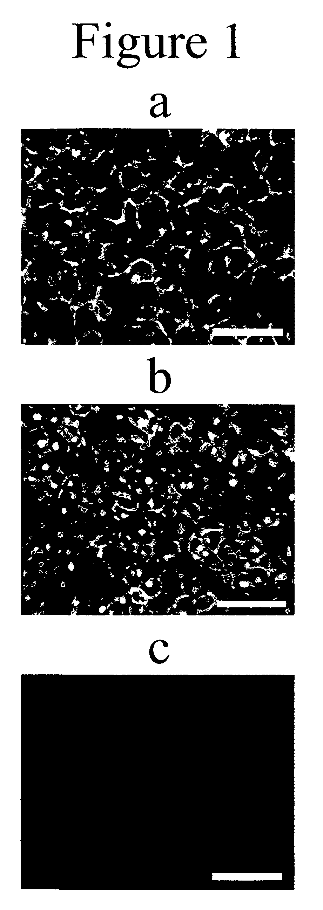

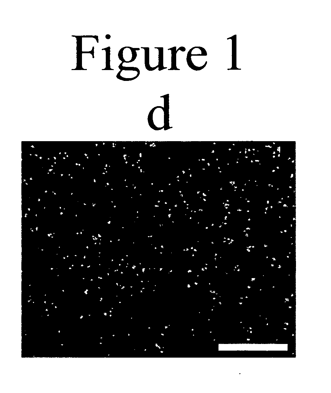

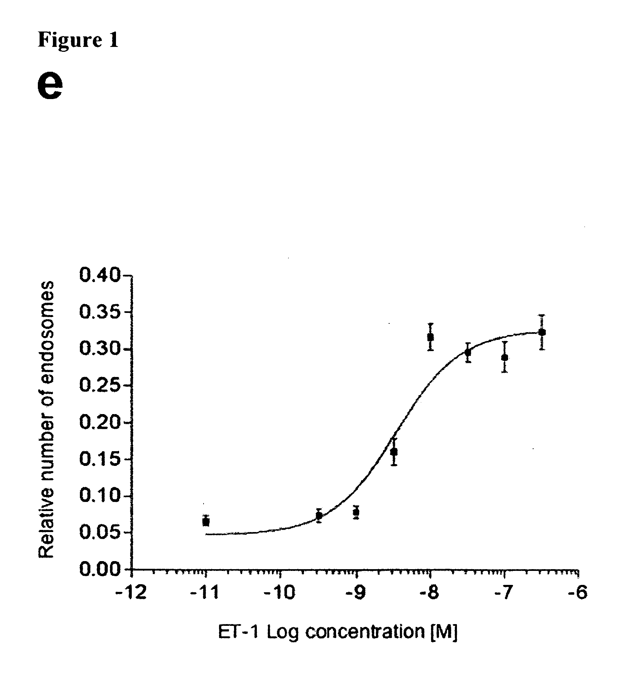

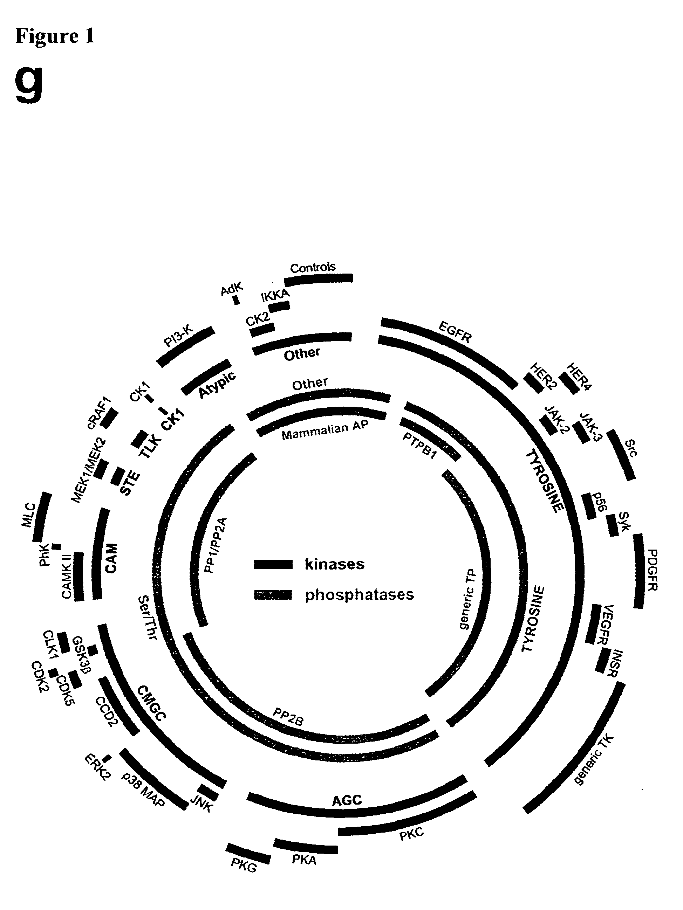

GPCR translocation was evaluated using scripts within the Acapella script software environment of the Opera. The script measured translocation of the ETAR-EGFP fusion protein from the plasma membrane into recycling endosomes. The script passed the acceptance criteria of measuring the EC₅₀ of endothelin stimulation as 3.4 nM, with a Z' factor of >0.7 (Fig. 1c). Curve fitting was performed with Graphpad Prism 4.0 for OS X. Housekeeping endocytosis was measured using custom-written scripts in Metamorph. Endocytosis was expressed as relative number of endosomes per cell, average endosomal pixel intensity and integrated total fluorescence intensity per cell. This assay had a Z' factor of 0.68.

### Ex vivo aortic contraction ex-vivo measurements

As previously described, male rats (Sprague-Dawley) weighing 300 to 400 g were anesthetized with sodium pentobarbital (50 mg/kg i.p.), and thoracic aorta was removed, cleaned of fat and connective tissue placed in Krebs bicarbonate solution (118mM NaCl; 4.7mM KC1; 25mM NaHCO₃; 2.5mM CaCl₂; 1.2 mM KH₂PO₄; 1.2mM MgSO₄ 11mM glucose) bubbled with 95% O₂ and 5% CO₂ ^{39, 40}. The whole aorta was cut along a close spiral to produce a strip of 5 to 6 cm long. The strip was mounted on a Grass force displacement transducer at a tension of 2 grams and placed in a 25 ml chamber maintained at 34°C. Following mounting of aorta strip on transducer, noradrenalin was added to a final concentration of 5µM and increase of tension-constriction was recorded for 20 min. Subsequently, the noradrenalin was washed with 16 chamber volumes (400ml) until tension returned to 2 grams. Each Drug (H89, Erbstatin A and Go6976) was added to a final concentration of 10µM and incubated for 30 min subsequent to which ET was added to a final concentration of 40nM and further incubated for another 30 minutes. Tension was recorded throughout the experiment. To enable comparative study between preparations data is shown relative to noradrenalin constriction. Each experiment was repeated at least 3 times.

### Results

The vasoactive agonist endothelin activates the coupling of the endothelin A receptor to Gq and Gs, causing vasoconstriction and also induces rapid internalization of the receptor. To identify compounds capable of arresting the internalization and resensitization (recycling cycle) of the GPCR, we used a high content screen of agonist-induced internalization to screen a palette of established kinase and phosphatase inhibitors. The involvement of kinases in the endocytic pathway has been described ²². Our rationale was to examine the sensitivity of both GPCR internalization to kinase inhibitors, and exclude those acting indirectly on the receptor by disrupting housekeeping endocytosis. To enable the correlation between compounds identified in the cell based assay and their physiological impact, we examined their effect in an ex vivo vasoconstriction model.

### Automated GPCR and endocytosis screens

Many G-protein coupled receptors are internalized via membrane traffic upon agonist exposure. Agonist induced Endothelin A receptor internalization was imaged in a HEK293 cell line stably expressing a fusion protein between the ETAR and the enhanced green fluorescent protein, while housekeeping endocytosis was resolved by imaging fluid phase fluorescent marker internalization.

The ETAR fusion protein was localized to the plasma membrane, with no detectable internal labeling (Fig. 1a) and showed agonist dependent internalization into an intracellular peri-centriolar recycling endosome (Fig. 1b), as confirmed by co localization with red fluorescent transferrin (data not shown,). Computational image analysis automatically determined the fraction of cells with the receptor in the pericentriolar recycling endosome (Fig. 1e). Computational analysis was applied to cells treated with a titration of endothelin-1 and data fitting determined an EC₅₀ of 3.4 nM endothelin-1 for agonist dependent ETAR activation (Fig. le), in agreement with reported measurements ²³⁻²⁵, a Z' factor > 0.7 ²⁶ and validated the assay as a measure of GPCR activation and internalization.

The housekeeping endocytosis assay measured internalization of a green fluorescent 40 kDa BODIPY-FL dextran. Dextrans are ideal inert markers for labeling endosomes and macropinosomes via fluid phase endocytosis because of their high solubility ²⁷. Endosomes labeled with fluorescent dextran were detected in living cells by automated confocal imaging after 20 minutes of internalization at 37°C (Fig. 1d). Dextran uptake was temperature dependent, arrested at 4°C (Fig. 1c), and required a source of energy in the medium (data not shown). Image analysis determined the relative number of endosomes per cell, the average pixel intensity of each endosome and the integrated intensity per cell. The analysis was validated on cells following internalization of fluorescent dextran for 20 min at 4°C or 37°C with a Z' factor >0.68 ²⁶. This assay measured housekeeping endocytic processes that contribute to ETAR endocytosis but that are not receptor specific- such as caveolar endocytosis, clathrin mediated endocytosis and macro-pinocytosis.

### Kinase inhibitor screen

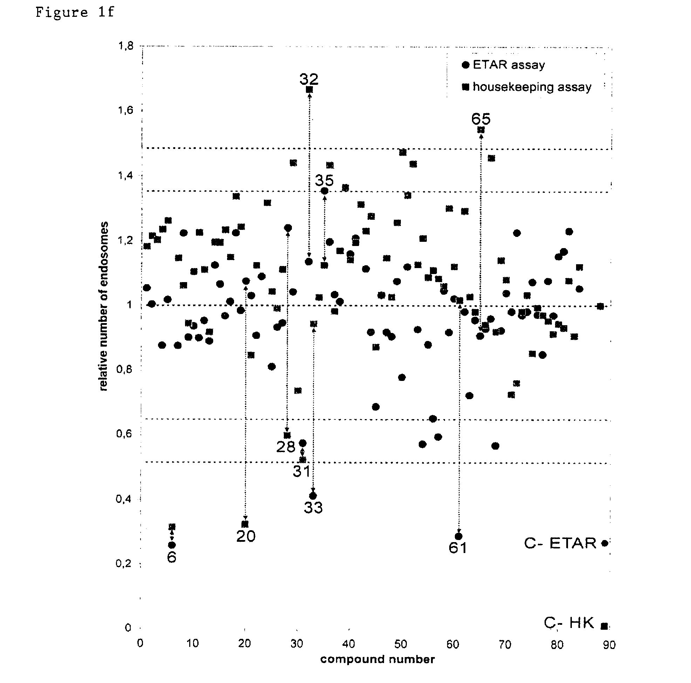

The ETAR and housekeeping visual screens were performed with a kinase and phosphatase inhibitor collection (n=84) by automated confocal imaging and computational image analysis. As shown in the rotary map in Figure 1g, the collection of inhibitors included molecules directed against kinases of eight out of nine group/families described in the recent classification of the human kinome and against three classes of phosphatases²⁸. ETAR expressing cells were treated with 10 µM compounds prior to and throughout agonist stimulation and then analyzed by automated imaging. No internalization was observed in unstimulated cells treated with DMSO alone (Fig. 1a). No changes in receptor distribution were detected after compound treatment prior to stimulation with agonist (data not shown). Internalization of the receptor in control-stimulated and control-unstimulated cells corresponded to the expected values from the endothelin-1 titration (Fig. 1c). Inhibitors were identified that significantly altered receptor internalization. Potent hits, such as staurosporine (compound_6, Fig 1f), were defined as those giving relative numbers of labeled endosomes outside a threshold of ± two-to-three times the standard deviation of the untreated but endothelin stimulated control. Lethal effects were excluded by visual examination and removed from the screen (see Supplementary data 1).

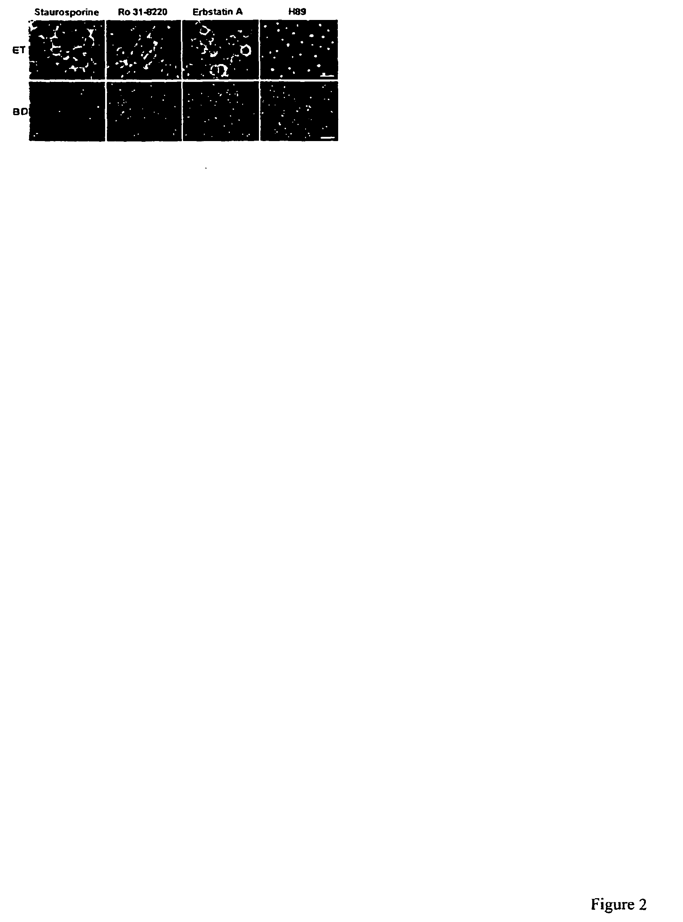

Five compounds affected agonist dependent ETAR endocytosis with three distinct phenotypes: ETAR arrest at the cell surface (Ro 31 8220, Palmitoyl DL carnitine chloride and staurosporine), ETAR arrest in peripheral early endosomes (erbstatin analogue) and ETAR accumulation in the recycling endosome (H-89) (Fig. 2). The compounds were classified according to the phenotype identified by visual analysis. (A complete list of tested compounds is provided in supplementary data 1). Four compounds affected housekeeping endocytosis (staurosporine, tyrphostin 9, AG-879, GF 109203X) and reduced the number of endosomes (Fig. 1g) and the integrated intensity per cell (data not shown). Visual analysis of the cells treated with the compounds did not identify any morphological anomalies compared to control cells, except the absence of internalized green fluorescent dextran. Of the 9 compounds affecting either the ETAR or the housekeeping assay, 3 only affecting the ETAR assay were identified by superimposing the GPCR and housekeeping screens (Fig If). Superimposition identified molecules whose effect was similar in both assays (e.g. staurosporine and GF 109203X; Fig. 1f) from those with different effects, such as the PKC bisindoylmaliemide inhibitor Ro 31-8220, the tyrosine kinase inhibitor erbstatin A, and the protein kinase A inhibitor H-89 (Fig If). PKC inhibition blocked the ETAR at the cell surface, tyrosine kinase inhibition blocked ETAR in peripheral endosomes and protein kinase A inhibition promoted receptor trafficking to the recycling endosome (Fig 2). None of these compounds had a measurable effect on the housekeeping endocytosis assay (Fig 2). In contrast, other compounds such as staurosporine (compound 6) - inhibited both ETAR and housekeeping endocytosis. A secondary screen was carried out on a subset of compounds including the hits of the primary screen to assign IC50's of 1µM for RO 31 8220, 2.5µM for Erbstatin A and 10µM for H-89 (Supplementary figure 2).

### Single cell visual screening

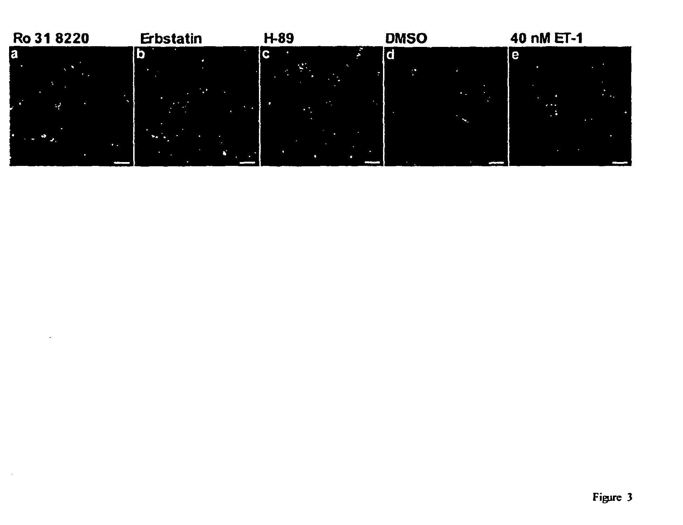

The effect of the 3 kinase inhibitors affecting ETAR on receptor activation and housekeeping endocytosis were simultaneously imaged in ETAR: GFP expressing cells by internalization of red fluorescent dextran (Fig 3). Cells pretreated with selected inhibitors followed by stimulation with ET-1 had dramatically altered internalization of the receptor with only marginal effects on internalization of the fluid phase marker (Fig. 3a-c). The protein kinase C inhibitor Ro 31-8220 arrested ETAR at the plasma membrane but housekeeping endocytosis was unaffected (Fig. 3a). The erbstatin analog arrested the ETAR at the plasma membrane and in endosomes but housekeeping endocytosis was unchanged (Fig 3b). The protein kinase A inhibitor H-89 increased delivery of the receptor to the recycling endosome but left housekeeping endocytosis unaltered (Fig. 3c). Thus, inhibition of protein kinase A and C second messenger activated kinases potently altered ETAR GPCR internalization but left housekeeping endocytosis unaffected.

### G-protein coupled receptor specificity of kinase inhibitors blocking ETAR trafficking

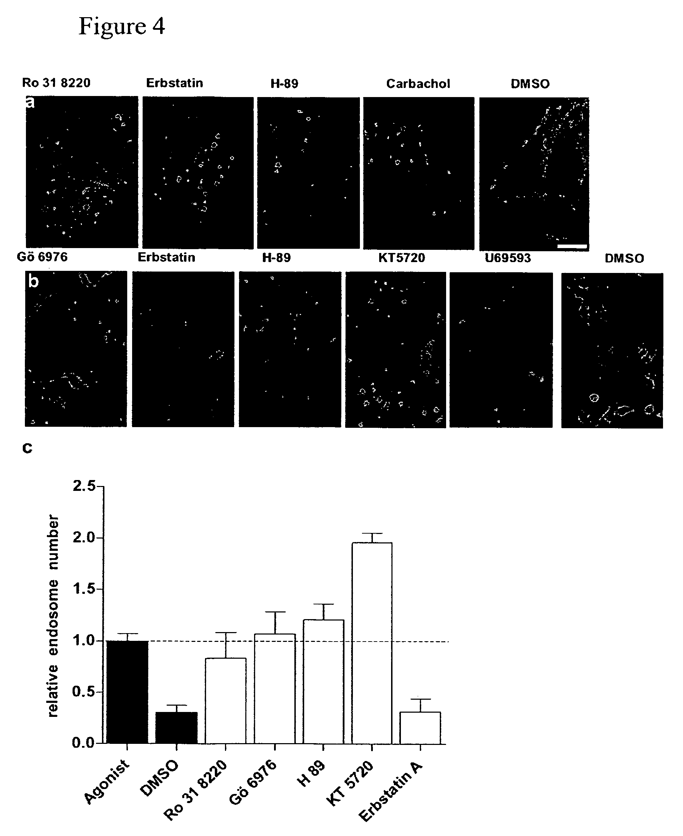

If the involvement of second messenger kinases in agonist induced endocytosis of the ETAR was a generic feature of Gq coupled receptor activation, it may be expected that the kinase inhibitors affecting ETAR endocytosis may have effects on related receptors or related receptors in other cell lines. The kinase inhibitors that disrupted agonist induced ETAR endocytosis were assayed on agonist-induced endocytosis of the Gq/11 coupled M1 muscarinic acetylcholine receptor (M1AR). Agonist-induced M1AR endocytosis was unaffected by 10µM RO 31 8220, 10µM Erbstatin A or 10µM H-89 in comparison to the untreated agonist stimulated positive control (Fig 4A). This demonstrated that the inhibitor targets are not part of the machinery required for the endocytosis of a similar Gq coupled GPCR.

To determine if the effect of the inhibitors was through the disruption of the GPCR trafficking machinery, we then examined compound effects on the internalization of a human kappa Opioid receptor fusion to the green fluorescent protein (kOPr). The kOPr undergoes agonist induced internalization endocytic trafficking and recycling (reviewed in ²⁹) and the kOPr-GFP fusion protein showed an EC50 of 300nM for agonist (U50488H, U69593) measured by image analysis of kOPr endocytosis to perinuclear endosomes. Protein kinase C inhibition did not affect receptor uptake, but erbstatin arrested the receptor in the periphery and protein kinase A inhibition with either a moderate (H-89) or pronounced stimulation of receptor uptake (KT-5720) (Fig 4B,C). Therefore, cell based assays of GPCR internalization were effective at the selection of at least one compound class that affects only endothelin A receptor internalization in the context of receptors tested thus far.

PKC inhibition blocks ETAR internalization while erbstatin or PKA inhibition may affect the GPCR endocytic machinery common to the ETAR and kOPr. Erbstatin can be defined as an inhibitor of transport to the peri-centriolar endosomes, and PKA inhibitors as affecting GPCR recycling, but protein kinase C inhibition was only effective on the ETAR.

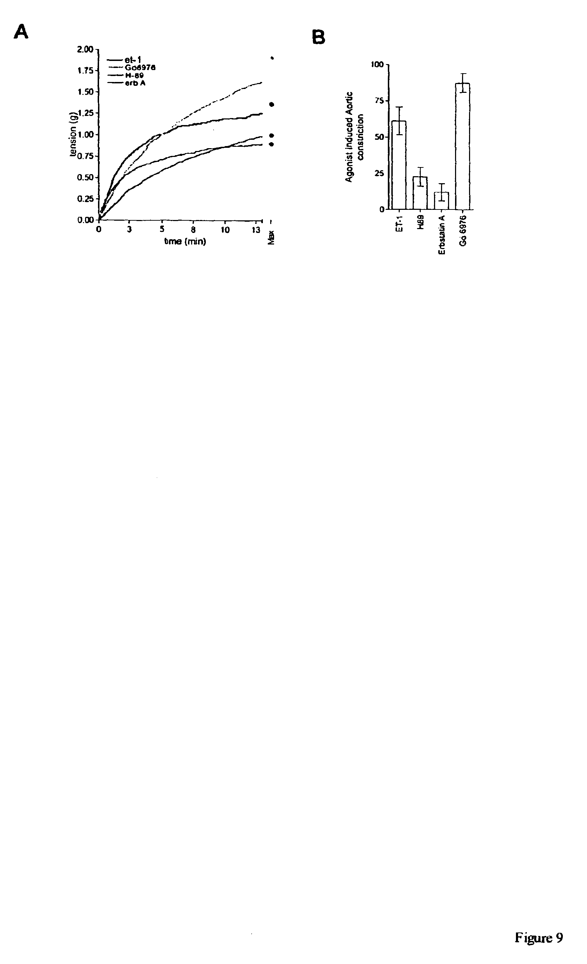

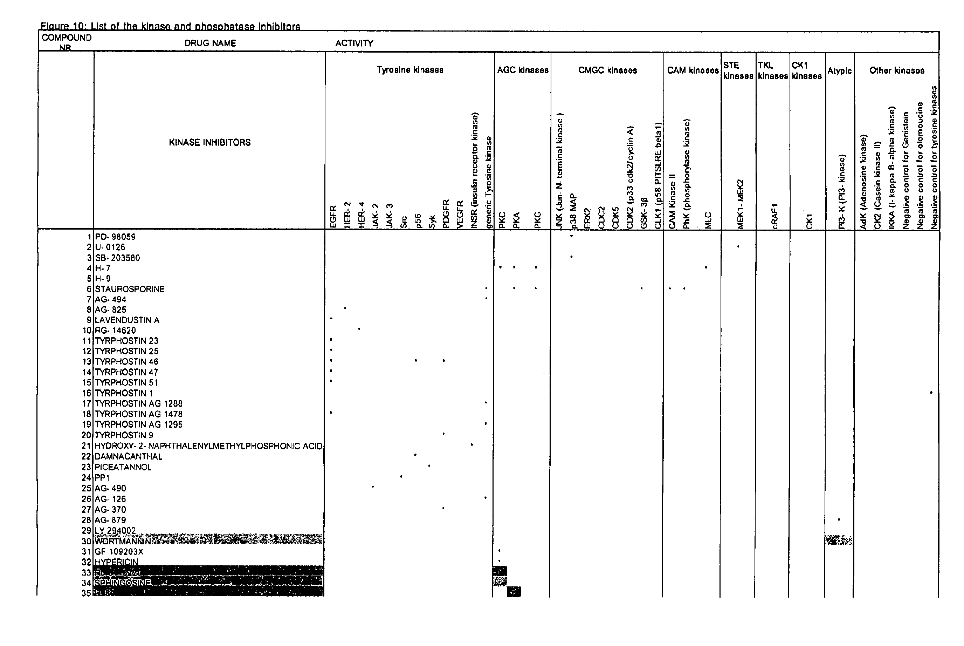

Therefore, compounds acting on a single receptor were identified using GPCR cell based assays, a starting point for compound optimization and large scale screening. While PKC inhibition is most selective toward the ETAR over M1AR and kOPr, the effect of disrupting GPCR transport on physiology was examined using Erbstatin and PKA inhibitor mediated disruption of trafficking (Figure 9).

### G-protein-activated Protein kinase C regulates ETAR internalization

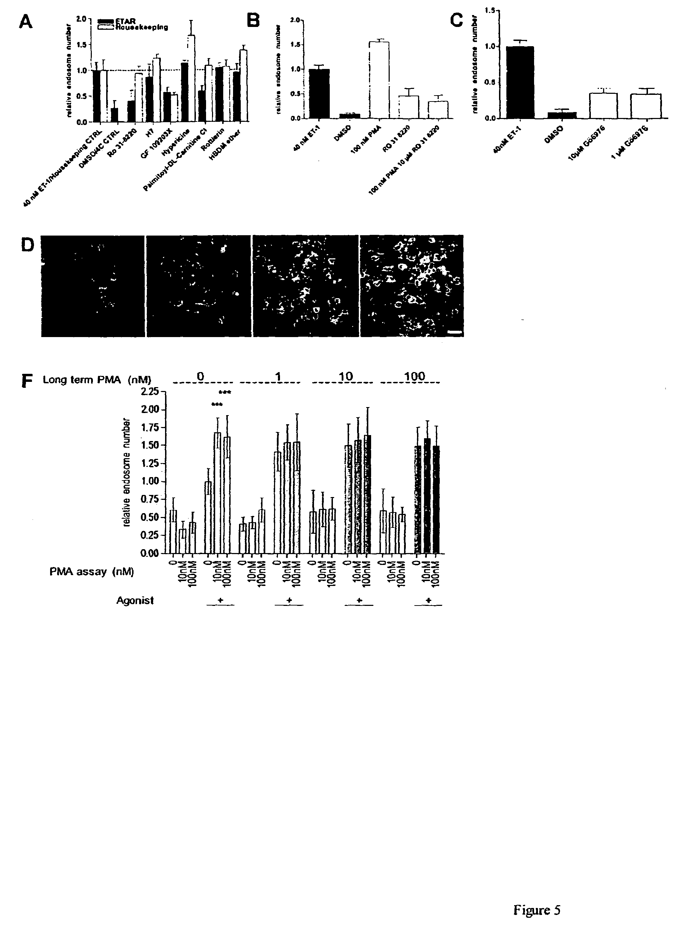

Visual screening indicated a role for protein kinase C in ETAR endocytosis from the cell surface (Fig 1,2). Following the guidelines for use of kinase inhibitors in cell based assays ^{30, 31} we examined the effect of a panel of structurally diverse Protein kinase C inhibitors on the ETAR and housekeeping endocytosis assays (Fig 5A). Inhibition of protein kinase C alpha and beta with Ro 31 8220, or the structurally unrelated Palmitoyl DL carnitine chloride reduced ETAR endocytosis (0.5 fold) had no significant effect on housekeeping endocytosis (Fig. 5a). The PKC delta-isoform specific inhibitor rottlerin had no effect on either assay, indicating that a subset of PKC isoforms control ETAR endocytosis. The PKC inhibitor GF109203x blocked both ETAR and housekeeping endocytosis, in contrast HDBM ether and H7 had no significant effect on the assays. Interestingly, hypericine stimulated housekeeping endocytosis 0.2 fold compared to control but had a marginal effect on ETAR internalization.

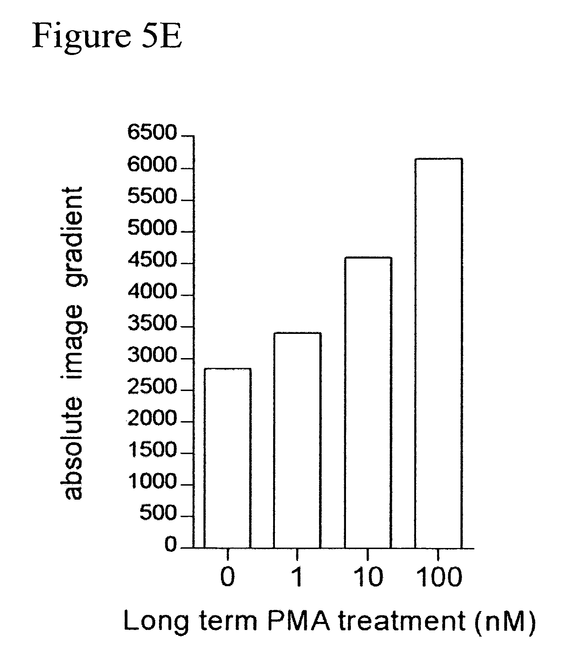

Phorbol ester stimulation of PKC activity (100nM PMA) increased ET-1 induced ETAR endocytosis by >50% (Fig. 5b), with no internalization in the absence of stimulation. The phorbol ester stimulation of ETAR internalization was reduced to the negative control level when cells were simultaneously treated with PMA and RO 31 8220 (Fig. 5b). This indicated that the PKC isoform(s) sensitive to Ro 31 8220 were activated by phorbol ester and were required for receptor internalization. ETAR internalization was also inhibited by Gö 6976 (1µM), a highly specific alpha and beta PKC isoform inhibitor ³² (Fig 5C), indicating the involvement of PKC a and b in the internalization of ETAR. Depletion of endogenous protein kinase C through long-term (24 hour, 100 nM) phorbol ester treatment increased the level of the ETAR at the cell surface 2.6 fold (Figure 5D & 5E). After washing out phorbol ester, endothelin stimulated internalization of the endothelin A receptor was no longer increased by phorbol ester treatment (Figure 5F) indicating that downregulated PKC is required for ETAR endocytosis.

### G-protein activated Protein kinase A regulates ETAR recycling

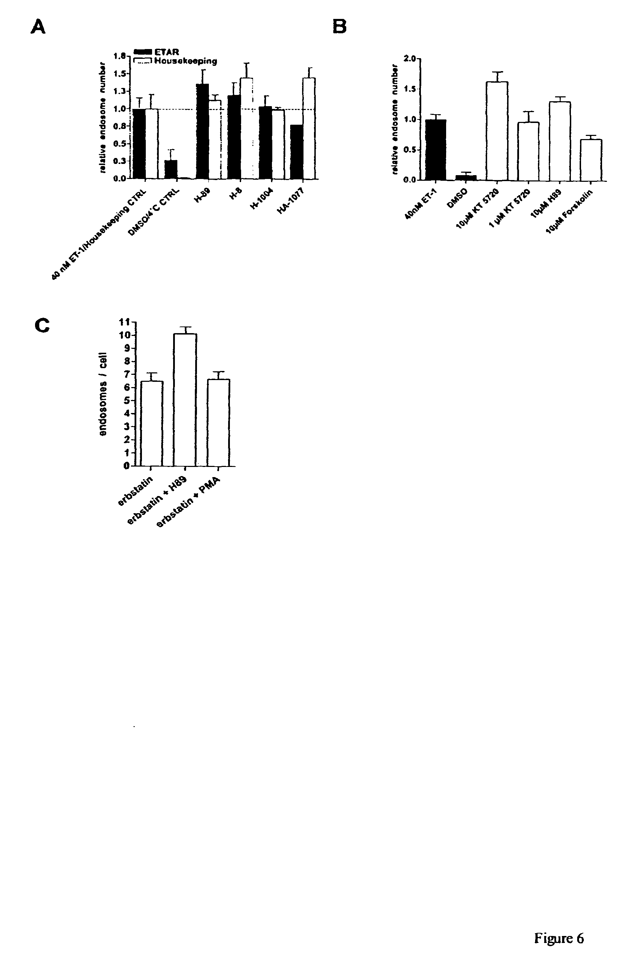

The protein kinase A inhibitor H-89 caused accumulation of the ETAR in the recycling endosome after stimulation. This corresponded to a 1.25 fold stimulation in ETAR internalization compared to control (Fig 6a). Stimulation of cAMP production with 10µM forskolin blocked the effect of H-89 (Fig. 6b). Treatment of cells with 10µM KT5720, a similar PKA inhibitor, stimulated ETAR sequestration >1.5 fold and the GPCR accumulated in the recycling endosome (Fig 6b).

We examined the effect of activating protein kinase A via adenyl cyclase activation by 10 µM forskolin on the EC50 for endothelin in the ETAR assay. Forskolin increased the EC50 from 3.4 to 7nM endothelin, whereas treatment of cells with H-89 increased the slope of the dose-response curve to ET-1 (supplementary figure 3). This indicated that PKA was required for either ETAR endocytosis or the regulation of ETAR recycling. PKA inhibition correlated with increased ETAR transport to the recycling endosome, which could be explained by a requirement for PKA in recycling to the cell surface from the endosome. To determine whether PKA was involved in recycling from the early endosome, we used either erbstatin A or microtubule disruption with nocodazole to prevent ETAR transport to the recycling endosome and measured the number of ETAR positive endosomes at the cell periphery. Image analysis showed that the number of endosomes per cell was comparable unless cells were co-treated with PKA inhibitors (H-89 or KT5720) when there was a 1.5 fold increase in the number of endosomes per cell (Fig 6c). Therefore, protein kinase A activity is either involved in regulating the number of GPCR positive endosomes (i.e. via fusion/fission) or transport of the ETAR from early endosomes to the cell surface, as seen for other GPCRs ³³.

### An erbstatin A sensitive tyrosine kinase regulates ETAR trafficking/sorting to the recycling endosome

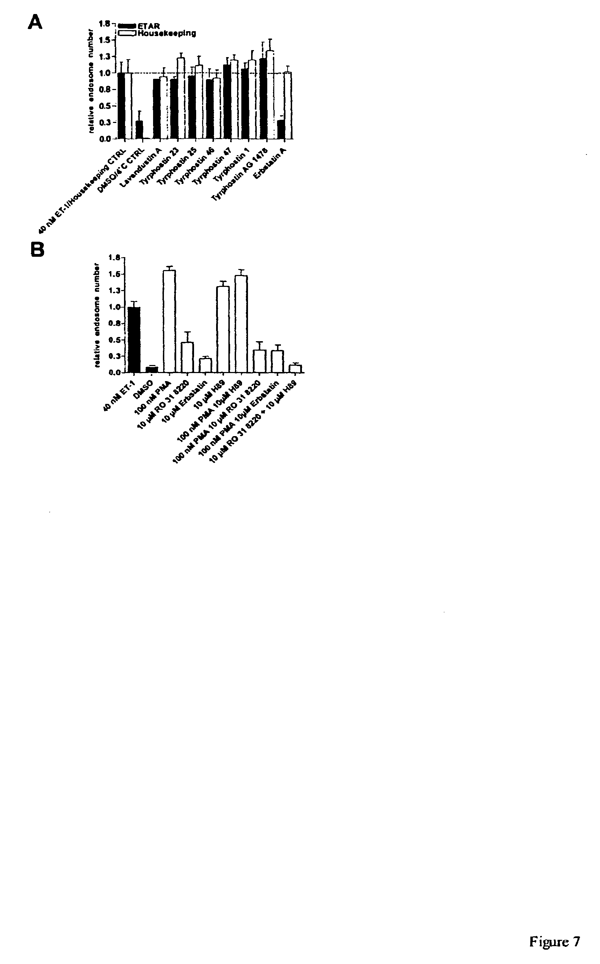

The visual screens identified erbstatin A as acting on the ETAR endocytic trafficking pathway. A panel of tyrosine kinase inhibitors were screened (Fig. 7) and of these only erbstatin A arrested ETAR in endosomes but had no effect on housekeeping endocytosis. When added to cells stimulated with PMA or where PKA was inhibited by H-89, erbstatin blocked receptor internalization to the recycling endosome (Fig 7b). Erbstatin has one known target kinase- the EGF receptor tyrosine kinase- and it has been shown that the ETAR trans-activates the EGFR (^{34, 35}). To assess if EGFR kinase activity played a role in ETAR transport to the recycling endosome, we measured ETAR endocytosis in the presence of 10-100 ng/mL EGF with or without ET-1 stimulation and erbstatin pretreatment. ETAR internalization was not affected by EGFR activation and this had no effect on the inhibition of ETAR transport by erbstatin A (not shown). It is likely that EGFR is not the erbstatin A sensitive kinase required for ETAR exit from the early endosome to the recycling endosome.

### GPCR activated PKC regulates ETAR endocytosis and receptor coupling to Gs

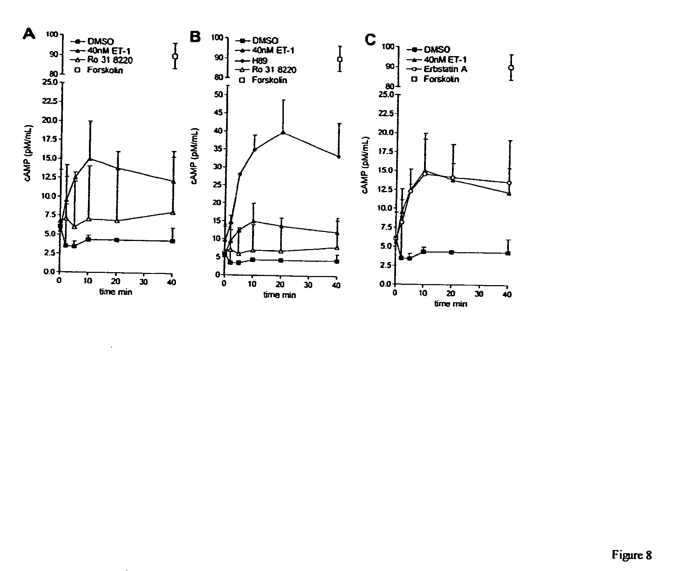

GPCRs can couple to multiple G-proteins and switching between G-proteins is generally mediated by protein phosphorylation ³⁶⁻³⁸. We assessed the importance of Gq coupling and PKC activity in the generation of intra-cellular cAMP through Gs following agonist stimulation. In cells stimulated with ET-1, there was a robust increase in intra-cellular cAMP after 10 min (Fig. 8a). Treatment of the cells with the PKC inhibitor Ro 31 8220 blocked cAMP production (Fig. 8a), whereas Erbstatin A had no effect compared to the control (Fig. 8c). In contrast, H-89 markedly increased cAMP over control (Fig. 8b). Gq coupling and Protein kinase C activation are a requirement for ETAR coupling to Gs, and in the presence of PKC inhibitors, agonist mediated ETAR activation does not lead to CAMP production. Restriction of ETAR to the recycling endosome with H-89 increased intracellular cAMP, indicating that internalization is required for cAMP production and recycling to the PM may terminate Gs coupling,

### Endothelin A receptor cellular antagonism modulates vasoconstriction

To correlate the effect of kinase inhibitors that perturb ETAR intracellular pathway and CAMP production to a physiological relevance we have examined compound effect(s) on the ex-vivo rat thoracic aorta constriction-relaxation model (^{39,40}). Aortal strips harboring both endothelial cells as well as smooth muscles were mounted on a mechanosensor submerged in gassed Krebs solution. Strips were pretreated with Noradrenalin to evaluate the extent of viability of the aorta to contract and to enable normalization of experiments. Subsequently, aorta was washed extensively for 15 minutes to relax the aorta. Contractile responses were assessed after aortal strips were treated with 10 µM Gö6976, 10µM Erbstatin A or 10µM H-89 for 30 minutes followed by treatment with Endothelin. In each experiment the drug-endothelin dependent contraction was normalized to the preceding noradrenalin contraction. As shown in figure 9, PKC inhibition with Go6976 gave increased contraction than controls treated with endothelin alone, with a similar slope but a significant increase in amplitude that was significantly increased over control (Figure 9a). PKA inhibition with H89 which we have shown to arrest the receptor at the recycling endosome and dramatically increased cAMP levels resulted in significantly lower constriction of the smooth muscle compared to treatment with endothelin alone, (Figure 9A, 9B). Treatment of aortal strips with Erbstatin A- which arrested ETAR in early endosomes in our cell based assay, also significantly inhibited muscle constriction. This may indicate that intracellular localization of the receptor promotes relaxation. It is initiated within the early endosomes and achieves its peak at the recycling endosomes.

This data consistent with a model where ETAR is prevented from internalization when PKC is inhibited and this leads to prolonged Gq activation and aortal contraction. In contrast, arrest of ETAR recycling with H-89 may reduce the constrictive response of aortal strips to endothelin by decreasing the pool of cell surface receptor as it accumulated in the recycling endosome.

This indicates the GPCR related physiology can be modulated by compounds that function as *'cellular antagonists'* of receptor function and are a novel approach to drug development for GPCRs.

### Discussion

The present data demonstrates a drug discovery approach relying on the identification of compounds perturbing the subcellular pathway of G-protein coupled receptors. High content screening of a cell based assay of agonist induced receptor internalization readily identified compounds that arrested the endothelin A receptor at the cell surface or in endosomes.

This identified the mechanism underlying the auto-regulation of receptor transport through signaling, but when these molecules were used to modulate endothelin physiology in the aorta model they proved to be effective. Receptor arrest at the cell surface lead to aortal super-constriction, and intracellular retention lead to lowered constriction, showing that the strategy of intervening in receptor : cell interaction rather than receptor : agonists interactions are effective in drug discovery.

An important aspect of using cell based assays to select drugs that cause phenotypic disruption of receptor trafficking is to exclude effects on the pathways underlying receptor trafficking. Compounds were excluded that had effects on ETAR internalization and also disrupted endocytosis - as assessed by measured alterations in the uptake of inert fluid phase dextran. To further demonstrate the potential for introducing specificity at the level of the receptor affected by compounds, we screened compounds affecting ETAR but not endocytosis on the agonist induced internalization of the muscarinic acetylcholine receptor 1 and the kappa opioid receptor. While endocytosis of the M1Ar was apparently unaffected kappa opioid receptor internalization was arrested by two of the three compound classes acting on the ETAR- erbstatin A and protein kinase A inhibitors. Interestingly protein kinase C inhibition only affected internalization of the ETAR.

We observed that stimulation of the Endothelin A-receptor coordinates endocytic sequestration of the receptor through the activation of the second messenger activated protein kinase C and protein kinase A. The activation of these kinases is triggered by agonist binding to the heptahelical receptor and receptor coupling to Gq and Gs proteins. Activation of protein kinase C was required for ETAR endocytosis from the cell surface and protein kinase A activity promoted receptor recycling to the cell surface.

The involvement of protein kinase C in ETAR internalization is inferred from the observations that PKC inhibition, with compounds specific for PKC alpha and beta isoforms, blocks agonist dependent internalization and that PKC activation with phorbol ester stimulates receptor endocytosis. Since phorbol ester stimulated ETAR internalization was agonist dependent and blocked by the PKC inhibitors, this may reflect the involvement of a classical, phorbol ester activated PKC in ETAR endocytosis. HEK293 cells express very low amounts of the PKC alpha isoform ⁴¹, it is also likely that the beta PKC isoforms are the target of compounds used here. Protein kinase C has been implicated in a variety of GPCR endocytosis mechanisms, but most significantly in the heterologous (receptor independent) desensitization of signaling for the 5HTA receptor. The sensitivity to Gö6976, which is highly specific for the alpha and beta isoforms ³², is consistent with the involvement of PKC-beta in ETAR internalization. The actual substrate for PKC phosphorylation is unclear, while there is evidence for direct receptor phosphorylation from in vivo labeling ⁴² and there are 7 consensus PKC phosphorylation sites in the cytoplasmic tail of the ETAR.

Inhibition of protein kinase A by either H-89 or the structurally unrelated inhibitor KT5720, caused the accumulation of the ETAR in the peri-centriolar recycling endosome. This is consistent with a role for the activation of protein kinase A in ETAR recycling via the receptor coupling to Gs. PKA has been implicated in recycling of the beta1-adrenergic receptor ³³ as is observed for the ETAR. Given the role of PKA, an erbstatin sensitive kinase and PKC in ETAR endocytosis, we analyzed the effects of their inhibition on cAMP production. cAMP generation was robust after endothelin stimulation with a peak in production 20 minutes following stimulation, as seen previously ⁴³, but was blocked by PKC inhibition. Arrest of ETAR in early endosome with erbstatin had no effect on cAMP generation, whereas PKA inhibition with H-89 increased cellular cAMP two-fold. This is consistent with a requirement for PKC activity for the delivery of ETAR to an endosomal compartment where it couples to Gs and activates adenyl cyclase. Gs internalization has been seen for activation of the beta2-adrenergic receptor ⁴⁴.

Ex vivo aortal strip measurements demonstrated that the arrest of ETAR transport seen in the cell model modulated the physiology of vasoconstriction. The endothelin dependent effect of the compounds on vasoconstriction indicated that PKC inhibition - which presumably arrested the endothelin A receptor at the cell surface- led to super-constriction of the aorta strips. ETAR coupling to Gq triggers increases in cytosolic calcium and chemical prevention of receptor internalization is consistent with a model where this prevents receptor uptake and thus potentiates constriction. In contrast, inhibition of ETAR recycling decreased contraction upon exposure to endothelin 1 which is consistent with reduced receptor recycling diminishing the pool of cell surface receptor available to couple to Gq. This is evidence that the endocytic cycling of ETAR coordinates aortal contraction and that the endocytic cycle of this receptor may participate in the regulation of contraction through receptor location in addition to the well established mechanisms invoking the roles of second messengers.

Chemical intervention into the signaling events regulating heptahelical-receptor-specific desensitization (homologous) and resensitization may be a route into new pharmaceuticals through increased understanding of the mechanism by which the agonists activate or inhibit receptors to regulate signaling networks and receptor location. There is evidence that disrupted receptor trafficking alters receptor related physiology, as in the case of the Vasopressin 2 receptor R137H mutation in nephrogenic diabetes insipidus {Bernier et al, 2004 Mol Endocrinol 18(8):2074-84; Barak et al, 2001 proc Natl Acad Sci USA 98(1):93-8} . Therefore, compounds altering receptor distribution could be attractive for regulating disease physiology.

### References

1. Pierce, K. L., Premont, R. T. & Lefkowitz, R. J. Seven-transmembrane receptors. Nat Rev Mol Cell Biol 3, 639-50 (2002).
2. Neves, S. R., Ram, P. T. & Iyengar, R. G protein pathways. Science 296, 1636-9 (2002).
3. Ross, E. M. & Gilman, A. G. Biochemical properties of hormone-sensitive adenylate cyclase. Annu Rev Biochem 49, 533-64 (1980).
4. Krupnick, J. G. & Benovic, J. L. The role of receptor kinases and arrestins in G protein-coupled receptor regulation. Annu Rev Pharmacol Toxicol 38, 289-319 (1998).
5. Pitcher, J. A., Freedman, N. J. & Lefkowitz, R. J. G protein-coupled receptor kinases. Annu Rev Biochem 67, 653-92 (1998).
6. Lefkowitz, R. J. & Whalen, E. J. beta-arrestins: traffic cops of cell signaling. Curr Opin Cell Biol 16, 162-8 (2004).
7. Shenoy, S. K. & Lefkowitz, R. J. Multifaceted roles of beta-arrestins in the regulation of seven-membrane-spanning receptor trafficking and signalling. Biochem J 375, 503-15 (2003).
8. Yanagisawa, M. et al. A novel potent vasoconstrictor peptide produced by vascular endothelial cells. Nature 332, 411-5 (1988).
9. Yoshizawa, T. et al. Endothelin: a novel peptide in the posterior pituitary system. Science 247, 462-4 (1990).
10. Sokolovsky, M. Endothelins and sarafotoxins: physiological regulation, receptor subtypes and transmembrane signaling. Trends Biochem Sci 16, 261-4 (1991).
11. Rubanyi, G. M. & Botelho, L. H. Endothelins. Faseb J 5, 2713-20 (1991).
12. Miller, R. C., Pelton, J. T. & Huggins, J. P. Endothelins--from receptors to medicine. Trends Pharmacol Sci 14, 54-60 (1993).
13. Abe, Y., Nakayama, K., Yamanaka, A., Sakurai, T. & Goto, K. Subtype-specific trafficking of endothelin receptors. J Biol Chem 275, 8664-71 (2000).
14. Bohm, F., Pernow, J., Lindstrom, J. & Ahlborg, G. ETA receptors mediate vasoconstriction, whereas ETB receptors clear endothelin-1 in the splanchnic and renal circulation of healthy men. Clin Sci (Lond) 104, 143-51 (2003).
15. Foster, N., Loi, T. H., Owe-Young, R. & Stanley, K. K. Lysosomal traffic of liganded endothelin B receptor. Biochim Biophys Acta 1642, 45-52 (2003).
16. Hackman, A. M. & Lackner, T. E. Pharmacotherapy for idiopathic pulmonary arterial hypertension during the past 25 years. Pharmacotherapy 26, 68-94 (2006).
17. Kamsteeg, E. J. et al. Reversed polarized delivery of an aquaporin-2 mutant causes dominant nephrogenic diabetes insipidus. J Cell Biol 163, 1099-109 (2003).
18. Marr, N. et al. Heteroligomerization of an Aquaporin-2 mutant with wild-type Aquaporin-2 and their misrouting to late endosomes/lysosomes explains dominant nephrogenic diabetes insipidus. Hum Mol Genet 11, 779-89 (2002).
19. Abraham, V. C., Taylor, D. L. & Haskins, J. R. High content screening applied to large-scale cell biology. Trends Biotechnol 22, 15-22 (2004).
20. Granas, C. et al. High content screening for G protein-coupled receptors using cell-based protein translocation assays. Comb Chem High Throughput Screen 8, 301-9 (2005).
21. Milligan, G. et al. G protein-coupled receptor fusion proteins in drug discovery. Curr Pharm Des 10, 1989-2001 (2004).
22. Pelkmans, L. et al. Genome-wide analysis of human kinases in clathrin- and caveolae/raft-mediated endocytosis. Nature 436, 78-86 (2005).
23. Wiley, K. E. & Davenport, A. P. Endothelin receptor pharmacology and function in the mouse: comparison with rat and man. J Cardiovasc Pharmacol 44 Suppl 1, S4-6 (2004).
24. Wu-Wong, J. R., Berg, C. E., Wang, J., Chiou, W. J. & Fissel, B. Endothelin stimulates glucose uptake and GLUT4 translocation via activation of endothelin ETA receptor in 3T3-L1 adipocytes. J Biol Chem 274, 8103-10 (1999).
25. Clark, K. L. & Pierre, L. Characterization of endothelin receptors in rat renal artery in vitro. Br J Pharmacol 114, 785-90 (1995).
26. Zhang, J. H., Chung, T. D. & Oldenburg, K. R. A Simple Statistical Parameter for Use in Evaluation and Validation of High Throughput Screening Assays. J Biomol Screen 4, 67-73 (1999).
27. Yamashiro, D. J. & Maxfield, F. R. Kinetics of endosome acidification in mutant and wild-type Chinese hamster ovary cells. J Cell Biol 105, 2713-21 (1987).
28. Manning, G., Whyte, D. B., Martinez, R., Hunter, T. & Sudarsanam, S. The protein kinase complement of the human genome. Science 298, 1912-34 (2002).
29. Liu-Chen, L. Y. Agonist-induced regulation and trafficking of kappa opioid receptors. Life Sci 75, 511-36 (2004).
30. Davies, S. P., Reddy, H., Caivano, M. & Cohen, P. Specificity and mechanism of action of some commonly used protein kinase inhibitors. Biochem J 351, 95-105 (2000).
31. Bain, J., McLauchlan, H., Elliott, M. & Cohen, P. The specificities of protein kinase inhibitors: an update. Biochem J 371, 199-204 (2003).
32. Martiny-Baron, G. et al. Selective inhibition of protein kinase C isozymes by the indolocarbazole Go 6976. J Biol Chem 268, 9194-7 (1993).
33. Gardner, L. A., Delos Santos, N. M., Matta, S. G., Whitt, M. A. & Bahouth, S. W. Role of the cyclic AMP-dependent protein kinase in homologous resensitization of the beta1-adrenergic receptor. J Biol Chem 279, 21135-43 (2004).
34. Prenzel, N. et al. EGF receptor transactivation by G-protein-coupled receptors requires metalloproteinase cleavage of proHB-EGF. Nature 402, 884-8 (1999).
35. Daub, H., Weiss, F. U., Wallasch, C. & Ullrich, A. Role of transactivation of the EGF receptor in signalling by G-protein-coupled receptors. Nature 379, 557-60 (1996).
36. Lefkowitz, R. J., Pierce, K. L. & Luttrell, L. M. Dancing with different partners: protein kinase a phosphorylation of seven membrane-spanning receptors regulates their G protein-coupling specificity. Mol Pharmacol 62, 971-4 (2002).
37. Martin, N. P., Whalen, E. J., Zamah, M. A., Pierce, K. L. & Lefkowitz, R. J. PKA-mediated phosphorylation of the beta1-adrenergic receptor promotes Gs/Gi switching. Cell Signal 16, 1397-403 (2004).
38. Daaka, Y., Luttrell, L. M. & Lefkowitz, R. J. Switching of the coupling of the beta2-adrenergic receptor to different G proteins by protein kinase A. Nature 390, 88-91 (1997).
39. Furchgott, R. F. & Zawadzki, J. V. The obligatory role of endothelial cells in the relaxation of arterial smooth muscle by acetylcholine. Nature 288, 373-6 (1980).
40. Furchgott, R. F. & Bhadrakom, S. Reactions of strips of rabbit aorta to epinephrine, isopropylarterenol, sodium nitrite and other drugs. J Pharmacol Exp Ther 108, 129-43 (1953).
41. Wagey, R., Hu, J., Pelech, S. L., Raymond, L. A. & Krieger, C. Modulation of NMDA-mediated excitotoxicity by protein kinase C. J Neurochem 78, 715-26 (2001).
42. Freedman, N. J. et al. Phosphorylation and desensitization of human endothelin A and B receptors. Evidence for G protein-coupled receptor kinase specificity. J Biol Chem 272, 17734-43 (1997).
43. Aramori, I. & Nakanishi, S. Coupling of two endothelin receptor subtypes to differing signal transduction in transfected Chinese hamster ovary cells. J Biol Chem 267, 12468-74 (1992).
44. Hynes, T. R., Mervine, S. M., Yost, E. A., Sabo, J. L. & Berlot, C. H. Live cell imaging of Gs and the beta2-adrenergic receptor demonstrates that both alphas and beta1gamma7 internalize upon stimulation and exhibit similar trafficking patterns that differ from that of the beta2-adrenergic receptor. J Biol Chem 279, 44101-12 (2004).

All publications mentioned in the above specification, and references cited in said publications, are herein incorporated by reference. Various modifications and variations of the described methods and system of the present invention will be apparent to those skilled in the art without departing from the scope and spirit of the present invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

## Claims

1. A method for identifying a compound that modifies the cellular distribution of a GPCR in a population of cells comprising:
a) taking a population of cells expressing a GPCR
b) incubating said cells with a candidate compound and with a ligand of the GPCR
c) determining distribution of said GPCR in cells treated according to step b) and comparing with distribution of GPCR in cells incubated with a ligand of the GPCR in the absence of the candidate compound;
wherein altered distribution in cells incubated with the candidate compound is indicative that the candidate compound modifies GPCR cellular distribution.

2. A method as claimed in claim 1 wherein the GPCR undergoes agonist induced internalization.

3. A method as claimed in claim 2 wherein the GPCR is a β-adrenergic, endothelin A or kappa opioid or µ opioid receptor or muscarinic acetylcholine receptor.

4. A method as claimed in any of claims 1 to 3 wherein the GPCR is labelled.

5. A method as claimed in claim 4 wherein the label is a fluorescent label.

6. A method as claimed in any of claims 1 to 5 wherein distribution of the GPCR is determined by microscopy including optical microscopy, confocal microscopy or automated microscopic screening methods.

7. A method as claimed in any of claims 1 to 6 wherein the distribution of GPCR is compared by measuring labelled GPCR at the cell surface, or in peripheral endosomes or the pericentriolar recycling endosome or internal compartments..

8. A method as claimed in any of claims 1 to 7 wherein a candidate compound that modifies the cellular distribution of a GPCR is one that gives relative numbers of labelled endosomes outside a threshold of +/- two to three times the standard deviation of the untreated but ligand treated control cells.

9. A method as claimed in any of claims 1 to 8 further comprising comparing the effect of the candidate compound on the distribution of said labelled GPCR with its effect on house keeping endocytosis.

10. A method as claimed in claim 9 wherein the method comprises:
a) incubating cells with a marker
b) incubating cells with a candidate compound
c) determining the distribution of the marker in cells incubated with a candidate compound and comparing with distribution of marker in cells not incubated with a candidate compound wherein altered location in cells incubated with a candidate compound is indicative of a compound that modifies house keeping endocytosis.

11. A method as claimed in claim 10 wherein the marker is labelled dextran.

12. A method as claimed in any of claims 8 to 11 wherein the determination of GPCR location and marker internalisation are carried out simultaneously.

13. A method as claimed in any of claims 1 to 12 wherein the GPCR ligand is a GPCR agonist.

14. A method as claimed in any of claims 1 to 13 wherein an increase in GPCR at the cell surface is indicative of a candidate compound for treating a GPCR related disease.

15. A method as claimed in any of claims 1 to 13 wherein an increase in intracellular GPCR is indicative of a candidate compound for treating a GPCR related disease.

16. A method as claimed in claim 15 wherein an increase in intracellular GPCR is indicative of a candidate compound for slowed aortal vasoconstriction and increased relaxation.

17. A method as claimed in any of the preceding claims wherein said method further comprises additional assays to confirm candidate compound activity.

18. A method as claimed in claim 17 wherein the additional assay is an assay for vasoconstriction.

19. A method as claimed in claim 18 wherein the assay for vasoconstriction is an ex-vivo rat thoracic aorta constriction-relaxation model.

20. A method of modulating GPCR distribution in a cell comprising administering an inhibitor of PKC or PKA.

21. A method of treating a GPCR related disease comprising administering a compound that modifies the cellular distribution of a GPCR.

22. A compound that modifies cellular distribution of a GPCR for use in the treatment of a GPCR related disease.

23. A compound as claimed in claim 22 which selected from the protein kinase C inhibitors RO 31 8220, Gö 6976, palmitoyl DL carnitine chloride, protein kinase A inhibitors KT 5720, H-89 and erbstatin A.

24. Use of a compound that modifies the cellular distribution of a GPCR in the preparation of a medicament for use in the treatment of a GPCR related disease.

25. A use as claimed in claim 21 wherein the compound is selected from the protein kinase C inhibitors RO 31 8220, Gö 6976, palmitoyl DL carnitine chloride, protein kinase A inhibitors KT 5720, H-89 and erbstatin A.

26. A use as claimed in claim 21 or claim 22 wherein the GPCR related disease is endothelin related pulmonary arterial hypertension.
